(19) Europäisches Patentamt
European Patent Office
Office européen des brevets

(11) EP 1 862 179 A1

(12) # EUROPEAN PATENT APPLICATION
published in accordance with Art. 158(3) EPC

(43) Date of publication:
**05.12.2007 Bulletin 2007/49**

(21) Application number: **06728638.5**

(22) Date of filing: **28.02.2006**

(51) Int Cl.:
***A61K 39/395*** (2006.01) ***A61K 31/18*** (2006.01)
***A61K 31/404*** (2006.01) ***A61K 31/381*** (2006.01)
***A61K 31/498*** (2006.01) ***A61K 31/64*** (2006.01)
***A61P 9/00*** (2006.01) ***A61P 35/00*** (2006.01)
***C07D 209/30*** (2006.01) ***C07D 209/42*** (2006.01)
***C07D 241/44*** (2006.01) ***C07D 333/34*** (2006.01)

(86) International application number:
**PCT/JP2006/304208**

(87) International publication number:
**WO 2006/090928 (31.08.2006 Gazette 2006/35)**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **28.02.2005 JP 2005054150**

(71) Applicant: **Eisai R&D Management Co., Ltd.**
**Tokyo 112-8088 (JP)**

(72) Inventors:
- **SEMBA, Taro,**
**Tsukuba Research Lab., Eisai Co. Ltd.**
**Tsukuba-shi,**
**Ibaraki 300-2635 (JP)**
- **HATA, Naoko**
**Tsukuba-shi,**
**Ibaraki 300-2635 (JP)**
- **OZAWA, Yoichi**
**Tsukuba-shi,**
**Ibaraki 300-2635 (JP)**
- **OWA, Takashi**
**Tsukuba-shi,**
**Ibaraki 300-2635 (JP)**

(74) Representative: **Woods, Geoffrey Corlett**
**J.A. KEMP & CO.**
**Gray's Inn**
**14 South Square**
**London WC1R 5JJ (GB)**

(54) # NOVEL USE OF SULFONAMIDE COMPOUND IN COMBINATION WITH ANGIOGENESIS INHIBITOR

(57) The present invention relates to a pharmaceutical composition, a kit and a method for treating cancer and/or a method for inhibiting angiogenesis, comprising a sulfonamide compound in combination with Bevacizumab.

Figure 1

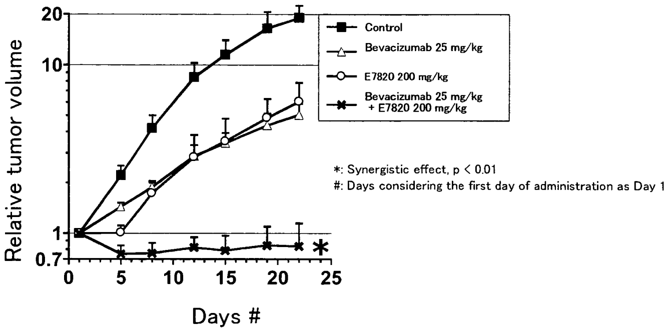


EP 1 862 179 A1

**Description**

FIELD OF THE INVENTION

The present invention relates to a novel pharmaceutical composition, a kit and a method for treating cancer and/or a method for inhibiting angiogenesis, characterized by comprising a sulfonamide compound in combination with Bevacizumab.

BACKGROUND OF THE INVENTION

Examples of conventionally used chemotherapy drugs for cancer include alkylating agents such as cyclophosphamide, antimetabolites such as methotrexate and fluorouracil, antibiotics such as adriamycin, mitomycin, bleomycin, plant-derived taxol, vincristine and etoposide, and metal complexes such as cisplatin. All of them, however, have not been sufficient in anti-tumor effects, and thus there has been a strong need for development of a novel anti-tumor agent.
Recently, a sulfonamide compound has been reported as a useful anti-tumor agent [1-5]. In particular, N-(3-chloro-1H-indole-7-yl)-4-sulfamoylbenzenesulfonamide (hereinafter, also referred to as "E7070"), N-(3-cyano-4-methyl-1H-indole-7-yl)-3-cyanobenzenesulfonamide (hereinafter, also referred to as "E7820"), N-[[(4-chlorophenyl)amino]carbonyl]-2,3-dihydro- 1H-indene-5-sulfbnamide (hereinafter, also referred to as "LY186641"), N-[[(3,4-dichlorophenyl)amino]carbonyl]-2,3-dihydrobenzofuran-5-sulfonamide (hereinafter, also referred to as "LY295501"), N-(2,4-dichlorobenzoyl)-4-chlorophenylsulfonamide (hereinafter, also referred to as "LY-ASAP"), N-(2,4-dichlorobenzoyl)-5-bromothiophene-2-sulfonamide (hereinafter, also referred to as "LY573636") and 2-sulfanylamide-5-chloroquinoxaline (hereinafter, also referred to as "CQS") are active against various types of tumors and thus are very useful.
On the other hand, as an antibody that inhibits angiogenesis, an anti-VEGF antibody Bevacizumab has been reported[6].
The presence and the kind of effect resulting from combining a sulfonamide compound and Bevacizumab, however, have not been reported so far. Although a combination of a sulfonamide compound and an anti-VEGF antibody has been reported to result in a synergistic effect, there is no mention of Bevacizumab[7].
Recently, methods were established for simultaneously detecting expression levels of multiple genes using various DNA microarrays. Thus, DNA microarrays have been used for wide-ranging purposes[8 and 9]. In addition, several reports have been made about using DNA microarrays (In part, there is a macroarray using membrane filters) for examining changes in gene expressions upon use of anti-cancer drugs against tumor cells (10-12) These reports show that the analysis of gene expression variability is highly useful in comprehensively studying the characteristic comparison among a plurality of cell populations, the biological changes in cells caused by treatment of drug or the like at molecular level.
Furthermore, reports have also been made to the analysis of gene expression profiles of 60 types of cancer cell line panels from the US National Cancer Institute for reclassification of these cell lines and examination of their characteristics[13], and to discussion regarding relationship among the gene expression profiles of these 60 types of cancer cell line panels and sensitivity of each cell line to various anti-cancer drugs[14].

References

(1) Japanese Laid-Open Patent Publication No. 7-165708.
(2) International Publication No. WO00/50395.
(3) European Patent Publication No. 0222475.
(4) International Publication No. WO02/098848.
(5) International Publication No. WO2003/035629.
(6) Direct evidence that the VEGF-specific antibody bevacizumab has antivascular effects in human rectal cancer. Nat Med. 2004 Feb:10 (2):145-7.
(7) International Publication No. WO03/074045.
(8) Schena M, Shalon D, Davis RW, Brown PO. Science, 1995, 270, 467-70.
(9) Lockhart, D.J., Dong, H., Byrne, M.C., Follettie, M.T., Gallo, M.V., Chee, M.S., Mittmann, M., Wang C., Kobayashi, M., Horton, H. Brown, E.L., Nature Biotechnology, 1996, 14, 1675-1680.
(10) Rhee CH, Ruan S, Chen S, Chenchik A, Levin VA, Yung AW, Fuller GN, Zhang W, Oncol Rep, 1999, 6, 393-401.
(11) Zimmermann J, Erdmann D, Lalande I, Grossenbacher R, Noorani M, Furst P, Oncogene, 2000, 19, 2913-20.
(12) Kudoh K, Ramanna M, Ravatn R, Elkahloun AG, Bittner ML, Meltzer PS, Trent JM, Dalton WS, Chin KV, Cancer Res, 2000, 4161-6.

(13) Ross DT, Scherf U, Eisen MB, Perou CM, Rees C, Spellman P, Iyer V, Jeffrey SS, Van de Rijn M, Waltham M, Pergamenschikov A, Lee JC, Lashkari D, Shalon D, Myers TG, Weinstein JN, Botstein D, Brown PO, Nat Genet, 2000, 24, 227-35.

(14) Scherf U, Ross DT, Waltham M, Smith LH, Lee JK, Tanabe L, Kohn KW, Reinhold WC, Myers TG, Andrews DT, Scudiero DA, Eisen MB, Sausville EA, Pommier Y, Botstein D, Brown PO, Weinstein JN, Nat Genet, 2000, 24, 236-44.

DISCLOSURE OF THE INVENTION

The present invention was achieved regarding the circumstances described above. The problem to be solved by the invention is to find a pharmaceutical composition and a kit having a remarkable anti-tumor activity and/or angiogenesis inhibitory activity, and a method for treating cancer and/or a method for inhibiting angiogenesis.

In order to solve the above problem, the present inventors have gone through keen examination, as a result of which combinational use of E7820 and Bevacizumab was found to show a statistically significant (by combination index) synergistic antiproliferative effect in a vascular endothelial cell proliferation assay (*in vitro*). In addition, combinational use of E7820 and Bevacizumab was found to show a statistically significant (by two-way ANOVA) synergistic anti-tumor effect in a subcutaneous transplant model *(in vivo)* of human colon cancer cell line. Moreover, combinational use of E7820 and Bevacizumab showed a remarkable anti-tumor effect that cannot be seen with Bevacizumab alone.

This combinational use of E7820 and Bevacizumab gave a remarkable synergistic effect as compared to combinational use of E7820 and an anti-VEGF antibody described in International Publication No. 03/074045 (pamphlet) (WO03/074045), which was unpredictable.

In addition, combinational use of E7070 and Bevacizumab was found to show a tendency for synergistic anti-tumor effect in a subcutaneous transplant model (*in vivo*) of human colon cancer cell line. Combinational use of E7070 and Bevacizumab further showed a remarkable anti-tumor effect that cannot be seen with Bevacizumab alone.

Moreover, combinational use of E7820 and Bevacizumab was found to show a statistically significant (by two-way ANOVA) synergistic anti-tumor effect in a subcutaneous transplant model *(in vivo)* of human renal cancer cell line. Combinational use of E7820 and Bevacizumab further showed a remarkable anti-tumor effect that cannot be seen with Bevacizumab alone.

In experiments using DNA microarrays and cancer cell line panels, genetic alteration patterns and antiproliferative activities of E7070, E7820, LY186641, LY295501, LY573636, CQS and combinations thereof were found to show high correlation. In an assay for determining antiproliferative activity, a cancer cell line resistant to E7070 was found to show cross-resistance to E7820, LY186641, LY295501, LY-ASAP, LY573636 or CQS. From these results, the present inventors have found that E7070, E7820, LY186641, LY295501, LY-ASAP, LY573636, CQS and combinations thereof have the same or similar action mechanisms that result in the same or similar genetic alterations and effects.

Accordingly, E7070, E7820, LY186641, LY295501, LY-ASAP, LY573636, CQS or a combination thereof is considered to show a good anti-tumor activity and angiogenesis inhibitory activity when used in combination with Bevacizumab, and thus a combination of a sulfonamide compound, preferably E7070, E7820, LY186641, LY295501, LY-ASAP, LY573636, CQS or a combination thereof, and Bevacizumab can be used as a useful pharmaceutical composition or a kit, and that they can be used for treatment of cancer and/or inhibition of angiogenesis.

Thus, the present invention relates to:

(1) A pharmaceutical composition comprising a sulfonamide compound in combination with Bevacizumab.

(2) A kit comprising:

- (a) at least one selected from the group consisting of a packaging container, an instruction and a package insert describing the combinational use of a sulfonamide compound and Bevacizumab; and
- (b) a pharmaceutical composition comprising the sulfonamide compound.

(3) A kit comprising a set of a formulation comprising a sulfonamide compound and a formulation comprising Bevacizumab.

(4) Use of a sulfonamide compound for producing a pharmaceutical composition in combination with Bevacizumab.

(5) A method for treating cancer and/or a method for inhibiting angiogenesis comprising administering a sulfonamide compound and Bevacizumab to a patient.

(6) A pharmaceutical composition comprising a sulfonamide compound for administering to a patient in combination with Bevacizumab.

The sulfonamide compounds according to (1)-(6) above include at least one compound selected from the group consisting of:

a compound represented by General Formula (I)

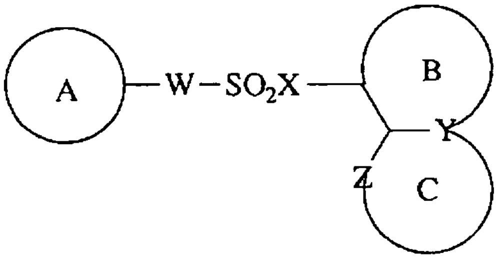

(I)

[wherein, ring A represents an optionally substituted monocyclic or bicyclic aromatic ring,
ring B represents an optionally substituted 6-membered cyclic unsaturated hydrocarbon or 6-membered unsaturated heterocycle containing a nitrogen atom as a heteroatom,
ring C represents an optionally substituted 5-membered heterocycle containing one or two nitrogen atoms,
W represents a single bond or -CH=CH-,
X represents $-N(R^1)-$ or an oxygen atom,
Y represents

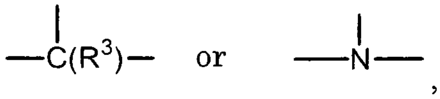

Z represents $-N(R^2)-$,
wherein, $R^1$, $R^2$ and $R^3$ independently represent, identically or differently, a hydrogen atom or a lower alkyl group];
a compound represented by General Formula (II)

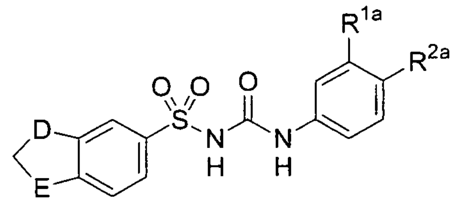

(II)

[wherein, E represents -O-, $-N(CH_3)-$, $-CH_2-$, $-CH_2CH_2-$ or $-CH_2O-$, D represents $-CH_2-$ or -O-, $R^{1a}$ represents a hydrogen atom or a halogen atom, and $R^{2a}$ represents a halogen atom or a trifluoromethyl group];
a compound represented by General Formula (III)

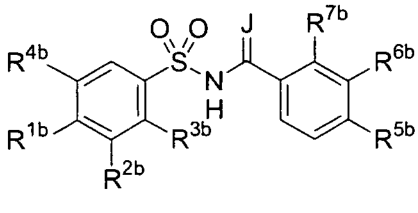

(III)

[wherein, J represents -O- or -NH-, $R^{1b}$ represents a hydrogen atom, a halogen atom, an optionally substituted $C_1$-$C_6$ alkyl group, an optionally substituted $C_1$-$C_4$ alkoxy group, an optionally substituted $C_1$-$C_4$ alkylthio group, $-CF_3$, $-OCF_3$, $-SCF_3$, an optionally substituted $C_1$-$C_4$ alkoxy carbonyl group, a nitro group, an azido group, $-O(SO_2)CH_3$, $-N(CH_3)_2$, a hydroxyl group, a phenyl group, a substituted phenyl group, a pyridinyl group, a thienyl group, a furyl group, a quinolinyl group or a triazole group, $R^{2b}$ represents a hydrogen atom, a halogen atom, a cyano group, $-CF_3$, an optionally substituted $C_1$-$C_6$ alkyl group, an optionally substituted $C_1$-$C_4$ alkoxy carbonyl group, an optionally

substituted $C_1$-$C_4$ alkoxy group, an optionally substituted phenyl group or an optionally substituted quinolinyl group, $R^{3b}$ represents a hydrogen atom or an optionally substituted $C_1$-$C_4$ alkoxy group, $R^{4b}$ represents a hydrogen atom or an optionally substituted $C_1$-$C_6$ alkyl group (provided that at least one of $R^{3b}$ and $R^{4b}$ is a hydrogen atom), $R^{5b}$ represents a hydrogen atom, a halogen atom, an optionally substituted $C_1$-$C_6$ alkyl group, $-CF_3$ or a nitro group, $R^{6b}$ represents a hydrogen atom, a halogen atom or an optionally substituted $C_1$-$C_6$ alkyl group (provided that when $R^{6b}$ is an optionally substituted $C_1$-$C_6$ alkyl group, $R^{5b}$ is a hydrogen atom and $R^{7b}$ is a halogen atom), $R^{7b}$ represents a halogen atom, an optionally substituted $C_1$-$C_6$ alkyl group or $-CF_3$ (provided that when either $R^{5b}$ or $R^{7b}$ is an optionally substituted $C_1$-$C_6$ alkyl group or when $R^{7b}$ is a halogen atom or an optionally substituted $C_1$-$C_6$ alkyl group, either $R^{5b}$ or $R^{6b}$ is a hydrogen atom)];
a compound represented by Formula (IV)

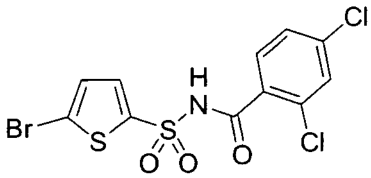

(IV)

;

and
a compound represented by Formula (V)

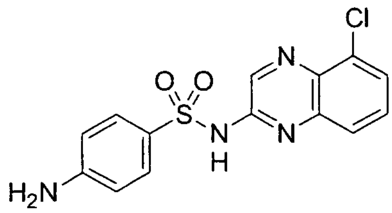

(V)

,

or a pharmacologically acceptable salt thereof, or a solvate thereof.

The present invention provides a pharmaceutical composition and a kit that show a remarkable anti-tumor activity and/or angiogenesis inhibitory activity, and a method for treating cancer and/or a method for inhibiting angiogenesis.

More specifically, the present invention provides a pharmaceutical composition and a kit that show a remarkable anti-tumor activity and/or angiogenesis inhibitory activity, and a method for treating cancer and/or a method for inhibiting angiogenesis, by combining a sulfonamide compound, that is, at least one compound selected from (A) a compound represented by General Formula (I), preferably E7070 or E7820, (B) a compound represented by General Formula (II), preferably LY186641 or LY295501, (C) a compound represented by General Formula (III), preferably LY-ASAP, (D) LY573636 and (E) CQS with Bevacizumab. Thus, the pharmaceutical composition, the kit and the methods of the invention can be used for cancer treatment or angiogenesis inhibition.

BRIEF DESCRIPTION OF THE DRAWINGS

Figure 1 shows an effect on tumor growth inhibition obtained by combinational use of E7820 and Bevacizumab in a subcutaneous transplant model *(in vivo)* of human colon cancer cell line (Colo320DM). In the figure, * indicates a statistically significant synergistic effect at a significance level of less than 0.01. In the figure, Day# indicates days from the first day of administration (Day 1).
Figure 2 shows the results of hierarchical cluster analysis in the DNA microarrays in Example 3.
Figure 3 shows correlation coefficients in the DNA microarrays in Example 4.
Figure 4 shows the results of hierarchical cluster analysis in the DNA microarrays in Example 4.
Figure 5 shows correlation coefficients in the DNA microarrays in Example 4.
Figure 6 shows the results of hierarchical cluster analysis in the DNA microarrays in Example 4.
Figure 7 shows gantiproliferative effects of E7070, E7820, CQS, LY186641, LY295501 and LY-ASAP on HCT116-C9, HCT116-C9-Cl and HCT116-C9-C4 as measured by cell growth inhibition assay.

Figure 8 shows antiproliferative effects of E7070 and LY573636 on HCT116-C9, HCT116-C9-C1 and HCT116-C9-C4 as measured by cell growth inhibition assay.

Figure 9 shows an effect on tumor growth inhibition obtained by combinational use of E7070 and Bevacizumab in a subcutaneous transplant model *(in vivo)* of human colon cancer cell line (Colo320DM).

BEST MODES FOR CARRYING OUT THE INVENTION

Hereinafter, embodiments of the present invention will be described. The following embodiments are described for illustrating the present invention and they are not intended to limit the present invention. The present invention may be carried out in various embodiments as long as it does not depart from the scope of the invention.

The publications, laid-open patent publications, patent publications and other patent documents cited herein are incorporated herein by reference.

1. Sulfonamide compound

A pharmaceutical composition and/or a kit, and a method for treating cancer and/or a method for inhibiting angiogenesis of the present invention comprise a sulfonamide compound.

According to the present invention, the sulfonamide compound comprises a compound represented by the following General Formula (I).

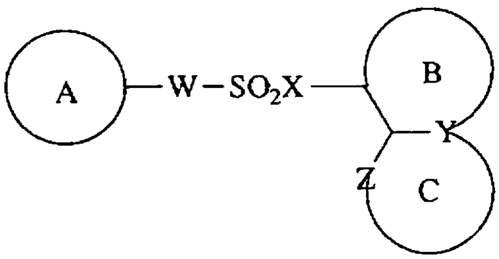

(I)

In General Formula (I),

ring A represents an optionally substituted monocyclic or bicyclic aromatic ring,
ring B represents an optionally substituted 6-membered cyclic unsaturated hydrocarbon or 6-membered unsaturated heterocycle containing a nitrogen atom as a heteroatom,
ring C represents an optionally substituted 5-membered heterocycle containing one or two nitrogen atoms,
W represents a single bond or -CH=CH-,
X represents $-N(R^1)-$ or an oxygen atom,
Y represents

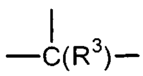 or 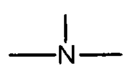 ,

Z represents $-N(R^2)-$.
$R^1$, $R^2$ and $R^3$ independently represent, identically or differently, a hydrogen atom or a lower alkyl group.

In General Formula (I), "an optionally substituted monocyclic or bicyclic aromatic ring" meant by ring A is an aromatic hydrocarbon or an aromatic heterocycle containing at least one of a nitrogen atom, an oxygen atom and a sulfur atom, which may have 1 to 3 substituents on the ring. Examples of the aromatic ring comprised in ring A mainly include pyrrole, pyrazole, imidazole, thiophene, furan, thiazole, oxazole, benzene, pyridine, pyrimidine, pyrazine, pyridazine, naphthalene, quinoline, isoquinoline, phthalazine, naphthyridine, quinoxaline, quinazoline, cinnoline, indole, isoindole, indolizine, indazole, benzofuran, benzothiophene, benzoxazole, benzimidazole, benzopyrazole and benzothiazole, although the aromatic ring comprised in ring A is not limited thereto. The aromatic ring may have 1 to 3 substituents, and when more than one substituent exist, they may be identical or different. Examples of the substituent

include an amino group that may be substituted with a lower alkyl group or a lower cyclo alkyl group, a lower alkyl group, a lower alkoxy group, a hydroxyl group, a nitro group, a mercapto group, a cyano group, a lower alkylthio group, a halogen atom, a group represented by Formula -a-b [wherein, a represents a single bond, $-(CH_2)_k-$, $-O-(CH_2)_k-$, $-S-(CH_2)_k-$ or $-N(R^3)-(CH_2)_k-$, k represents an integer of 1-5, $R^3$ refers to a hydrogen atom or a lower alkyl group, b represents $-CH_2-d$ (wherein, d represents an amino group that may be substituted with a lower alkyl group, a halogen atom, a hydroxyl group, a lower alkylthio group, a cyano group or a lower alkoxy group)], a group represented by Formula- a-e-f [wherein, a is as stated above, e represents -S(O)- or $-S(O)_2-$, f represents an amino group that may be substituted with a lower alkyl group or a lower alkoxy group, a lower alkyl group, a trifluoromethyl group, $-(CH_2)_m-b$ or $-N(R^4)-(CH_2)_m-b$ (wherein, b is as stated above, $R^4$ represents a hydrogen atom or a lower alkyl group, and m represents an integer of 1-5)], a group represented by Formula -a-g-h [wherein, a is as stated above, and g represents -C(O)- or -C(S)-, h represents an amino group that may be substituted with a lower alkyl group, a hydroxyl group, a lower alkyl group, a lower alkoxy group, $-(CH_2)_n-b$ or $-N(R^5)-(CH_2)_n-b$ (wherein, b is as stated above, $R^5$ represents a hydrogen atom or a lower alkyl group, and n represents an integer of 1-5)], a group represented by Formula $-a-N(R^6)-g-i$ [wherein, a and g are as stated above, $R^6$ represents a hydrogen atom or a lower alkyl group, and i represents a hydrogen atom, a lower alkoxy group or f (f is as stated above)], a group represented by Formula $-a-N(R^7)-e-f$ (wherein, a, e and f are as stated above, and $R^7$ refers to a hydrogen atom or a lower alkyl group), and a group represented by Formula $-(CH_2)_p-j-(CH_2)_q-b$ (wherein, j represents an oxygen atom or a sulfur atom, b is as stated above, and p and q identically or differently represent an integer of 1-5).

Among the exemplary substituents mentioned above, when the amino group is substituted with two alkyl groups, these alkyl groups may bind to each other to form a 5 or 6-membered ring. When ring A is a nitrogen-containing heterocycle having a hydroxyl group or a mercapto group, these groups may take a resonance structure and form an oxo group or a thioxo group.

In General Formula (I), "an optionally substituted 6-membered cyclic unsaturated hydrocarbon or 6-membered unsaturated heterocycle containing a nitrogen atom as a heteroatom" meant by ring B, for example, is benzene or pyridine in which a part of the unsaturated binding may be hydrogenated, which may have one or two substituents on the ring. When two or more substituents exist, they may be identical or different.

"An optionally substituted 5-membered heterocycle containing one or two nitrogen atoms" meant by ring C is pyrrole, pyrazole or imidazole in which a part of the unsaturated binding may be hydrogenated, which may have one or two substituents on the ring. When two or more substituents exist, they may be identical or different.

In General Formula (I), Z represents $-N(R^2)-$. $R^2$ and $R^1$ independently represent, identically or differently, a hydrogen atom or a lower alkyl group.

Examples of substituents that rings B and C may have include but not limited to a halogen atom, a cyano group, a lower alkyl group, a lower alkoxy group, a hydroxyl group, an oxo group, Formula -C(O)-r (wherein, r represents a hydrogen atom, an amino group that may be substituted with a lower alkyl group, a lower alkyl group, a lower alkoxy group or a hydroxyl group), an amino group that may be substituted with a lower alkyl group and a trifluoromethyl group.

In General Formula (I), Y represents

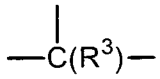 or 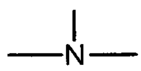

(wherein $R^3$ represents a hydrogen atom or a lower alkyl group).

In General Formula (I), "lower alkyl group" in the definition of the substituents thai $R^1$, $R^2$, $R^3$, ring A, ring B and ring C may have refers to a linear or branched alkyl group with a carbon number of 1-6, for example, but not limited to, a methyl group, an ethyl group, a n-propyl group, an isopropyl group, a n-butyl group, an isobutyl group, a sec-butyl group, a tert-butyl group, a n-pentyl group (an amyl group), an isopentyl group, a neopentyl group, a tert-pentyl group, a 1-methylbutyl group, a 2-methylbutyl group, a 1,2-dimethylpropyl group, a n-hexyl group, an isohexyl group, a 1-methylpentyl group, a 2-methylpentyl group, a 3-methylpentyl group, a 1-ethylpropyl group, a 1,1-dimethylbutyl group, a 1,2-dimethylbutyl group, a 2,2-dimethylbutyl group, a 1,3-dimethylbutyl group, a 2,3-dimethylbutyl group, a 3,3-dimethylbutyl group, a 1-ethylbutyl group, a 2-ethylbutyl group, a 1,1,2-trimethylpropyl group, a 1,2,2-trimethylpropyl group, a 1-ethyl-1-methylpropyl group and a 1-ethyl-2-methylpropyl group. Among these, examples of preferable group include a methyl group, an ethyl group, a n-propyl group, an isopropyl group, a n-butyl group, an isobutyl group, a sec-butyl group and a tert-butyl group, while examples of the most preferable group include a methyl group, an ethyl group, a n-propyl group and an isopropyl group.

The "lower cyclo alkyl group" in the definition of the substituent that ring A may have refers to a cyclo alkyl group with a carbon number of 3-8, for example, but not limited to, a cyclopropyl group, a cyclobutyl group, a cyclopentyl

group, a cyclohexyl group, a cycloheptyl group and a cyclooctyl group. The "lower alkylthio group" also refers to an alkylthio group derived from the lower alkyl group, for example, but not limited to, a methylthio group, an ethylthio group, a n-propylthio group, an isopropylthio group, a n-butylthio group, an isobutylthio group, a sec-butylthio group and a tert-butylthio group.

The "lower alkoxy group" in the definition of the substituents that ring A, ring B and ring C may have, for example, refers to, but not limited to, a lower alkoxy group derived from a lower alkyl group such as a methoxy group, an ethoxy group, a n-propoxy group, an isopropoxy group, a n-butoxy group, an isobutoxy group, a sec-butoxy group and a tert-butoxy group, the most preferable group being a methoxy group and an ethoxy group. In addition, examples of a "halogen atom" include a fluorine atom, a chlorine atom, a bromine atom and an iodine atom.

The compound represented by General Formula (I) of the invention can be produced according to a known method, for example, by those described in International Publication No. 95/07276 (pamphlet) (WO95/07276) and/or Japanese Laid-Open Patent Publication No. 7-165708 (JP7-165708).

In General Formula (I), a preferable compound is E7070 or E7820.

E7070 is N-(3-chloro-1H-indole-7-yl)-4-sulfamoylbenzenesulfonamide, whose structural formula is represented by the following Formula (VI).

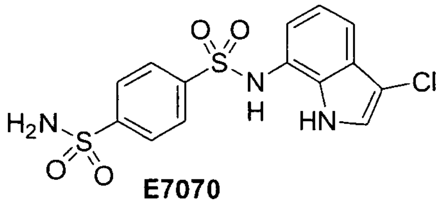

(VI)

**E7070**

E7070 can be produced according to a known method, for example, by those described in International Publication No. 95/07276 (pamphlet) (WO95/07276) and/or Example 19 of Japanese Laid-Open Patent Publication No. 7-165708 (JP7-165708).

E7820 is N-(3-cyano-4-methyl-1H-indole-7-yl)-3-cyanobenzenesulfonamide, whose structural formula is represented by the following Formula (VII).

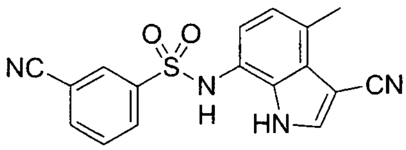

(VII)

**E7820**

E7820 can be produced according to a known method, for example, by a method described in International Publication No. 00/50395 (pamphlet) (WO00/50395).

According to the present invention, the sulfonamide compound comprises a compound represented by the following General Formula (II).

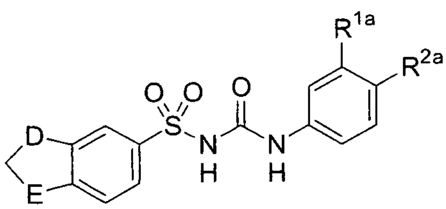

(II)

In General Formula (II) above, E represents -O-, $-N(CH_3)-$, $-CH_2-$, $-CH_2CH_2-$ or $-CH_2O-$, D represents $-CH_2-$ or -O-, $R^{1a}$ represents a hydrogen atom or a halogen atom (e.g., a fluorine atom, a chlorine atom, a bromine atom or an iodine atom), and $R^{2a}$ represents a halogen atom or a trifluoromethyl group.

The compound represented by General Formula (II) of the invention can be produced according to a known

method, for example, by a method described in European Patent Publication No. 0222475A1 (specification) (EP0222475A1).

In General Formula (II), a preferable compound is LY186641 or LY295501.

LY186641 is N-[[(4-chlorophenyl)amino]carbonyl]-2,3-dihydro-1H-indene-5-sulfonamide, whose structural formula is represented by the following Formula (VIII).

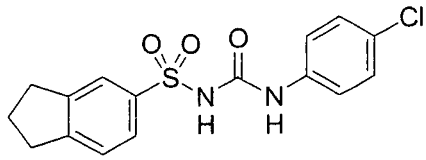

(VIII)

**LY186641**

LY186641 can be produced according to a known method, for example, by a method described in European Patent Publication No. 0222475A1 (specification) (EP0222475A1).

According to the present invention, LY295501 is N-[[(3,4-dichlorophenyl)amino]carbonyl]-2,3-dihydrobenzofuran-5-sulfonamide, whose structural formula is represented by the following Formula (IX).

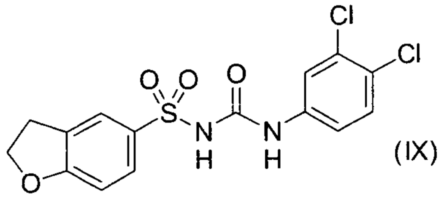

(IX)

**LY295501**

LY295501 can be produced according to a known method, for example, by those described in European Patent Publication No. 0222475A1 (specification) (EP0222475A1) and/or European Patent Publication No. 0555036A2 (specification) (EP0555036A2).

Furthermore, according to the present invention, the sulfonamide compound comprises a compound represented by the following General Formula (III).

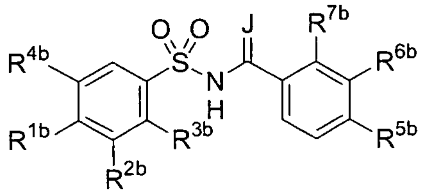

(III)

In General Formula (III), J represents -O- or -NH-, $R^{1b}$ represents a hydrogen atom, a halogen atom, an optionally substituted $C_1$-$C_6$ alkyl group, an optionally substituted $C_1$-$C_4$ alkoxy group, an optionally substituted $C_1$-$C_4$ alkylthio group, $-CF_3$, $-OCF_3$, $-SCF_3$, an optionally substituted $C_1$-$C_4$ alkoxy carbonyl group, a nitro group, an azido group, $-O(SO_2)CH_3$, $-N(CH_3)_2$, a hydroxyl group, a phenyl group, a substituted phenyl group, a pyridinyl group, a thienyl group, a furyl group, a quinolinyl group or a triazole group, $R^{2b}$ represents a hydrogen atom, a halogen atom, a cyano group, $-CF_3$, an optionally substituted $C_1$-$C_6$ alkyl group, an optionally substituted $C_1$-$C_4$ alkoxy carbonyl group, an optionally substituted $C_1$-$C_4$ alkoxy group, an optionally substituted phenyl group or an optionally substituted quinolinyl group, $R^{3b}$ represents a hydrogen atom or an optionally substituted $C_1$-$C_4$ alkoxy group, $R^{4b}$ represents a hydrogen atom or an optionally substituted $C_1$-$C_6$ alkyl group (provided that at least one of $R^{3b}$ and $R^{4b}$ is a hydrogen atom), $R^{5b}$ refers to a hydrogen atom, a halogen atom, an optionally substituted $C_1$-$C_6$ alkyl group, $-CF_3$ or a nitro group, $R^{6b}$ refers to a hydrogen atom, a halogen atom or an optionally substituted $C_1$-$C_6$ alkyl group (provided that when $R^{6b}$ is an optionally substituted $C_1$-$C_6$ alkyl group, $R^{5b}$ is a hydrogen atom and $R^{7b}$ is a halogen atom), $R^{7b}$ refers to a halogen atom, an optionally substituted $C_1$-$C_6$ alkyl group or $-CF_3$ (provided that when either $R^{5b}$ or $R^{7b}$ is an optionally substituted $C_1$-$C_6$ alkyl group or when $R^{7b}$ is a halogen atom or an optionally substituted $C_1$-$C_6$ alkyl group, either $R^{5b}$ or $R^{6b}$ is a hydrogen

atom).

In General Formula (III), a "halogen atom" is preferably a fluorine atom, a chlorine atom, a bromine atom or an iodine atom.

In General Formula (III), "$C_1$-$C_6$ alkyl group" is synonymous with the "lower alkyl group" described above, and preferably includes, but not limited to, a methyl group, an ethyl group, a n-propyl group, an isopropyl group, a n-butyl group, an isobutyl group, a sec-butyl group, a tert-butyl group, a n-pentyl group and a n-hexyl group.

In General Formula (III), "$C_1$-$C_4$ alkoxy group" refers to an alkoxy group with a carbon number of 1-4 of the "lower alkoxy groups" described above, and preferably includes, but not limited to, a methoxy group, an ethoxy group, a n-propoxy group, an isopropoxy group, a n-butoxy group, an isobutoxy group, a sec-butoxy group and a tert-butoxy group.

In General Formula (III), examples of alkyl group of "$C_1$-$C_4$ alkylthio group" include, but not limited to, methyl, ethyl, n-propyl, isopropyl, n-butyl, isobutyl, sec-butyl and tert-butyl.

In General Formula (III), examples of "$C_1$-$C_4$ alkoxy carbonyl group" include, but not limited to, a methoxy carbonyl group, an ethoxy carbonyl group, a n-propoxy carbonyl group, an isopropoxy carbonyl group, a n-butoxy carbonyl group, an isobutoxy carbonyl group, a sec-butoxy carbonyl group and a tert-butoxy carbonyl group.

In General Formula (III), examples of substituents to be introduced include, but not limited to, substituents such as a $C_1$-$C_6$ alkyl group (e.g., a methyl group, an ethyl group, a n-propyl group, an isopropyl group, a n-butyl group, an isobutyl group, a sec-butyl group, a tert-butyl group, etc.), a $C_1$-$C_4$ alkoxy group (e.g., a methoxy group, an ethoxy group, a n-propoxy group, an isopropoxy group, a n-butoxy group, an isobutoxy group, a sec-butoxy group, a tert-butoxy group, etc.), an amino group, a hydroxyl group, a halogen atom (e.g., a fluorine atom, a chlorine atom, a bromine atom or an iodine atom) and a silyl group.

The compound represented by General Formula (III) of the invention can be produced by a known method such as the method described in International Publication No. 02/098848 (pamphlet) (WO02/098848).

In General Formula (III), a preferable compound is LY-ASAP.

LY-ASAP is N-(2,4-dichlorobenzoyl)-4-chlorophenylsulfonamide, whose structural formula is represented by the following Formula (X).

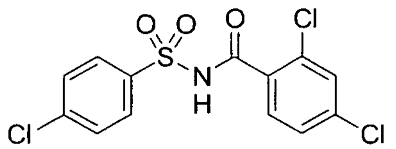

(X)

**LY-ASAP**

LY-ASAP can be produced by a known method such as the method described in International Publication No. 02/098848 (pamphlet) (WO02/098848).

According to the present invention, an example of the sulfonamide compound includes LY573636. According to the invention, LY573636 is N-(2,4-dichlorobenzoyl)-5-bromothiophene-2-sulfonamide, whose structural formula is represented by the following Formula (IV).

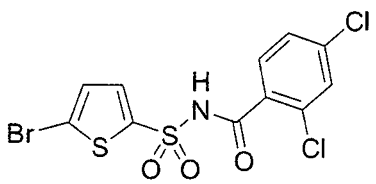

(IV)

LY573636

LY573636 is preferably in sodium salt form.

LY573636 can be produced by a known method. For example, it can be produced in the same manner as the method described in International Publication No. 02/098848 (pamphlet) (WO02/098848) using commercially available 5-bromothiophene-2-sulfonyl chloride and 2,4-dichlorobenzoic acid.

LY573636 can also be produced by a method described in Example 63 of International Publication No. 2003/035629 (pamphlet) (WO2003/035629).

According to the present invention, the sulfonamide compound may be CQS. According to the present invention, CQS is 2-sulfanylamide-5-chloroquinoxaline, whose structural formula is represented by the following Formula (V).

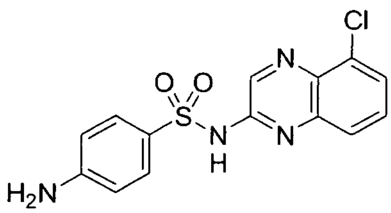

(V)

**CQS**

CQS can be produced according to a known method, for example, by a method described in (J. Am. Chem. Soc., 1947, 71, 6-10).

The sulfonamide compound may form a pharmacologically acceptable salt with acid or base. The sulfonamide compound of the invention also comprises these pharmacologically acceptable salts. Examples of salts formed with acids include inorganic acid salts such as hydrochloride salts, hydrobromide salts, sulfate salts and phosphate salts, and salts formed with organic acids such as formic acid, acetic acid, lactic acid, succinic acid, fumaric acid, maleic acid, citric acid, tartaric acid, benzoic acid, methanesulfonic acid, benzenesulfonic acid, p-toluenesulfonic acid and trifluoroacetic acid. Examples of salts formed with bases include alkali metal salts such as sodium salt and potassium salt, alkaline earth metal salts such as calcium salt and magnesium salt, salts with organic bases such as trimethylamine, triethylamine, pyridine, picoline, dicyclohexylamine, N,N'-dibenzylethylenediamine, arginine and lysine (organic amine salts), and ammonium salts.

Furthermore, the sulfonamide compound may be in anhydride form, and may form a solvate such as a hydrate. The solvate may be either a hydrate or a nonhydrate, preferably a hydrate. The solvent used may be water, alcohol (e.g., methanol, ethanol or n-propanol), dimethylformamide or the like.

If solvates and/or enantiomers of these compounds exist, the sulfonamide compound of the invention comprises these solvates and/or enantiomers. The sulfonamide compound of the invention also comprises a sulfonamide compound that undergoes metabolism such as oxidation, reduction, hydrolysis and conjugation *in vivo.* Moreover, the sulfonamide compound of the invention also comprises compounds that generate a sulfonamide compound by undergoing metabolism such as oxidation, reduction and hydrolysis *in vivo.*

2. Bevacizumab

A pharmaceutical composition and/or a kit, and a method for treating cancer and/or a method for inhibiting angiogenesis of the invention comprise Bevacizumab. Bevacizumab is a human anti-VEGF (Vascular Endothelial Growth Factor) monoclonal antibody and can be obtained by purchasing Avastin® from Genentech.

3. Pharmaceutical composition, kit, method for treating cancer and method for inhibiting angiogenesis

The present invention relates to a pharmaceutical composition, a kit, a method for treating cancer and a method for inhibiting angiogenesis, characterized by comprising a sulfonamide compound in combination with Bevacizumab.

According to the present invention, a sulfonamide compound is as described in "1. Sulfonamide compound". For example, the sulfonamide compound is at least one compound selected from: (A) a compound represented by General Formula (I), preferably E7070 or E7820; (B) a compound represented by General Formula (II), preferably LY186641 or LY295501; (C) a compound represented by General Formula (III), preferably LY-ASAP; (D) LY573636 (Formula (IV)) and (E) CQS (Formula (V)). Particularly preferably, the sulfonamide compound is at least one compound selected from LY295501 and LY573636 and more preferably sodium salt of LY573636.

According to the present invention, a sulfonamide compound is preferably E7070 or E7820.

According to the present invention, the sulfonamide compound and Bevacizumab also comprise pharmacologically acceptable salts thereof, or solvates such as hydrates thereof.

The pharmaceutical composition of the invention comprises a sulfonamide compound in combination with Bevacizumab. The pharmaceutical composition of the invention is useful for treating cancer and/or for inhibiting angiogenesis.

According to the present invention, the term "in combination with" refers to a combination of compounds for combinational use, and includes both modes in which separate compounds are administered in combination and as a mixture.

The pharmaceutical composition of the invention is also provided in another embodiment of a pharmaceutical composition comprising a sulfonamide compound, which is administered to a patient in combination with Bevacizumab. The sulfonamide compound and Bevacizumab may be administered either simultaneously or separately. The term "simultaneous" refers to administrations at the same timing in a single administration schedule. In this case, it is not necessary to use completely the same hour and minute for administration. The term "separately" refers to administrations at different timings in a single administration schedule.

The kit of the invention comprises a set of a formulation comprising a sulfonamide compound and a formulation comprising Bevacizumab. The formulations comprised in the kit of the invention are not limited to a particular form as long as they comprise a sulfonamide compound or Bevacizumab. The kit of the invention is useful for treating cancer and/or for inhibiting angiogenesis.

Furthermore, Bevacizumab contained in the pharmaceutical composition or kit of the invention may be a preparation available under the trade name of Avastin®.

In the kit of the invention, the formulation comprising a sulfonamide compound and the formulation comprising Bevacizumab may be mixed together or separately accommodated in a single package. They may be administered simultaneously or one may be administered preceding the other.

The pharmaceutical composition and/or the kit, and the method for treating cancer and/or the method for inhibiting angiogenesis of the invention may be further combined with one or more additional anti-cancer drugs. The additional anti-cancer drugs are not particularly limited as long as they are formulations having an anti-tumor activity. Examples of the additional anti-cancer drugs include irinotecan hydrochloride (CPT-11), oxaliplatin, 5-fluorouracil (5-FU), docetaxel (Taxotere®), gemcitabine hydrochloride (Gemzar®), calcium folinate (Leucovorin), Gefitinib (Iressa®), Erlotinib (Tarceva®) and Cetuximab (Erbitux®). Particularly preferable additional anti-cancer drugs are irinotecan hydrochloride, oxaliplatin, 5-fluorouracil, calcium folinate, Gefitinib, Erlotinib or Cetuximab when the type of cancer to be treated by the drug is colon cancer, gemcitabine hydrochloride, Gefitinib, Erlotinib or Cetuximab for pancreas cancer, and Gefitinib, Erlotinib or Cetuximab for renal cancer.

More examples of particularly preferable combinations of the compounds according to the invention are shown in Tables 1, 2 and 3 for the cases of treating colon cancer, pancreas cancer and renal cancer by the therapeutic drug, respectively.

Table 1

| | Combined Compounds | | | | | |
|---|---|---|---|---|---|---|
| 1 | E7070 | Bevacizumab | 5-FU | LV | Oxaliplatin | |
| 2 | E7820 | Bevacizumab | 5-FU | LV | Oxaliplatin | |
| 3 | E7070 | Bevacizumab | 5-FU | LV | Oxaliplatin | Gefitinib |
| 4 | E7820 | Bevacizumab | 5-FU | LV | Oxaliplatin | Gefitinib |
| 5 | E7070 | Bevacizumab | 5-FU | LV | Oxaliplatin | Erlotinib |
| 6 | E7820 | Bevacizumab | 5-FU | LV | Oxaliplatin | Erlotinib |
| 7 | E7070 | Bevacizumab | 5-FU | LV | Oxaliplatin | Cetuximab |
| 8 | E7820 | Bevacizumab | 5-FU | LV | Oxaliplatin | Cetuximab |
| 9 | E7070 | Bevacizumab | 5-FU | LV | CPT-11 | |
| 10 | E7820 | Bevacizumab | 5-FU | LV | CPT-11 | |
| 11 | E7070 | Bevacizumab | 5-FU | LV | CPT-11 | Gefitinib |
| 12 | E7820 | Bevacizumab | 5-FU | LV | CPT-11 | Gefitinib |
| 13 | E7070 | Bevacizumab | 5-FU | LV | CPT-11 | Erlotinib |
| 14 | E7820 | Bevacizumab | 5-FU | LV | CPT-11 | Erlotinib |
| 15 | E7070 | Bevacizumab | 5-FU | LV | CPT-11 | Cetuximab |
| 16 | E7820 | Bevacizumab | 5-FU | LV | CPT-11 | Cetuximab |
| 17 | E7070 | Bevacizumab | Gefitinib | | | |
| 18 | E7820 | Bevacizumab | Gefitinib | | | |
| 19 | E7070 | Bevacizumab | Erlotinib | | | |
| 20 | E7820 | Bevacizumab | Erlotinib | | | |
| 21 | E7070 | Bevacizumab | Cetuximab | | | |
| 22 | E7820 | Bevacizumab | Cetuximab | | | |

Table 1 shows preferable combinations of the invention where the type of cancer to be treated by the therapeutic

drug for cnacer is colon cancer. In the table, LV represents calcium folinate.

Table 2

| | Combined Compounds | | | |
|---|---|---|---|---|
| 1 | E7070 | Bevacizumab | Gemcitabine | |
| 2 | E7820 | Bevacizumab | Gemcitabine | |
| 3 | E7070 | Bevacizumab | Gemcitabine | Gefitinib |
| 4 | E7820 | Bevacizumab | Gemcitabine | Gefitinib |
| 5 | E7070 | Bevacizumab | Gemcitabine | Erlotinib |
| 6 | E7820 | Bevacizumab | Gemcitabine | Erlotinib |
| 7 | E7070 | Bevacizumab | Gemcitabine | Cetuximab |
| 8 | E7820 | Bevacizumab | Gemcitabine | Cetuximab |

Table 2 shows preferable combinations of the invention where the type of cancer to be treated by the therapeutic drug for cancer is pancreas cancer. In the table, Gemcitabine represents gemcitabine hydrochloride.

Table 3

| | Combined Compounds | | |
|---|---|---|---|
| 1 | E7070 | Bevacizumab | Gefitinib |
| 2 | E7820 | Bevacizumab | Gefitinib |
| 3 | E7070 | Bevacizumab | Erlotinib |
| 4 | E7820 | Bevacizumab | Erlotinib |
| 5 | E7070 | Bevacizumab | Cetuximab |
| 6 | E7820 | Bevacizumab | Cetuximab |

Table 3 shows preferable combinations of the invention where the type of cancer to be treated by the therapeutic drug for cancer is renal cancer.

The pharmaceutical composition and/or the kit of the invention can be used as a therapeutic drug for cancer and/or as an angiogenesis inhibitor.

Treatments according to the present invention comprise symptomatic relief of the disease, progression delay of symptoms of the disease, elimination of the symptoms of the disease, improvement of prognosis of the disease, and prevention of recurrence of the disease.

A therapeutic drug for cancer according to the invention comprises those that contain an anti-tumor agent, a drug for improving prognosis of cancer, a drug for preventing cancer recurrence, an antimetastatic drug or the like.

The effect of cancer treatment can be confirmed by observation of X-ray pictures, CT or the like, histopathologic diagnosis by biopsy, or tumor marker value.

The pharmaceutical composition and/or the kit of the invention can be administered to mammals (e.g., human, rat, rabbit, sheep, pig, cattle, cat, dog and monkey).

Examples of the types of cancers targeted by the therapeutic drug for cancer include, but not limited to, at least one selected from the group consisting of brain tumor, cervical cancer, esophageal cancer, tongue cancer, lung cancer, breast cancer, pancreas cancer, gastric cancer, small intestinal and duodenal cancer, colon cancer (colon cancer and rectal cancer), bladder cancer, renal cancer, liver cancer, prostate cancer, uterine cancer, ovarian cancer, thyroid grand cancer, gallbladder cancer, pharyngeal cancer, sarcoma (e.g., osteosarcoma, chondrosarcoma, Kaposi's sarcoma, myosarcoma, angiosarcoma, fibrosarcoma, etc.), leukemia (e.g., chronic myelocytic leukemia (CML), acute myelocytic leukemia (AML), chronic lymphocytic leukemia (CLL), acute lymphocytic leukemia (ALL), lymphoma, multiple myeloma (MM), etc.) and melanoma. Preferably, the type of cancer targeted by the therapeutic drug for cancer is at least one selected from the group consisting of pancreas cancer, renal cancer and colon cancer, and more preferably colon cancer.

The pharmaceutical composition and/or the kit of the invention may be administered orally or parenterally.

When the pharmaceutical composition and/or kit of the invention is used, the given dose of the sulfonamide compound differs depending on the degree of the symptom, age, sex, weight and sensitivity difference of the patient, administration mode, administration period, administration interval, and nature, prescription and type of the pharmaceutical formation and the type of the active ingredient. Usually, but without limitation, the dose of the sulfonamide compound is 10-6000 mg/day, preferably 50-4000 mg/day, more preferably 50-2000 mg/day for an adult (weight 60 Kg), which may be administered once to three times a day.

When the pharmaceutical composition and/or kit of the invention is used, the given dose of Bevacizumab is usually, but without limitation, 10-6000 mg/day, preferably 50-4000 mg/day, more preferably 50-2000 mg/day for an adult, which may be administered once to three times a day.

The amount of the sulfonamide compound used is not particularly limited, and differs depending on the individual combination with Bevacizumab. For example, the amount of the sulfonamide compound is about 0.01-100 times (weight ratio), more preferably about 0.1-10 times (weight ratio) of the amount of Bevacizumab.

The pharmaceutical composition of the invention may be made into various dosage forms, for example, into solid oral formulations or parenteral formulations such as rejection, suppository, ointment and skin patch.

Furthermore, the sulfonamide compound and Bevacizumab contained in the kit of the invention may individually be made into various dosage forms, for example, into solid oral formulations or parenteral formulations such as injection, suppository, ointment and skin patch.

In order to prepare a solid oral formulation, an excipient, and if necessary, a binder, disintegrant, lubricant, colorant, a flavoring agent or the like may be added to a principal agent, and then made into a tablet, a coated tablet, granule, subtle granule, powder, a capsule or the like according to a conventional method. In addition, a non-solid oral formulation such as a syrup agent can also be prepared appropriately.

For example, lactose, cornstarch, sucrose, glucose, sorbit, crystalline cellulose, silicon dioxide or the like may be used as the excipient; for example, polyvinyl alcohol, ethyl cellulose, methyl cellulose, gum arabic, hydroxypropyl cellulose, hydroxypropylmethyl cellulose or the like may be used as the binder; for example, magnesium stearate, talc, silica or the like may be used as the lubricant; those that are allowed to be added to pharmaceutical preparations may be used as the colorant; and for example, cocoa powder, menthol, aromatic acid, peppermint oil, camphor, cinnamon powder or the like may be used as the flavoring agent. Of course, if necessary, these tablets and granule may be coated appropriately with sugar coating, gelatin coating or else.

When an injection is to be prepared, if necessary, the principal agent may be added with a pH adjuster, a buffer, a suspending agent, a solubilizing aid, a stabilizer, an isotonizing agent, a preservative or the like, and may be made into an intravenously, subcutaneously or intramuscularly injection or an intravenous dorip injection according to a conventional method. In the case, if necessary, it is also prepared a lyophilized form by a conventional technique.

Examples of the suspending agent may include methyl cellulose, Polysorbate 80, hydroxyethyl cellulose, gum arabic, powdered tragacanth, sodium carboxy methyl cellulose and polyoxyethylene sorbitan monolaurate.

Examples of the solubilizing aid may include polyoxyethylene hydrogenated castor oil, Polysorbate 80, nicotine acid amide, polyoxyethylene sorbitan monolaurate, macrogol, and ethyl ester of castor oil fatty acid.

Examples of the stabilizer may include sodium sulfite and sodium metasulfite; examples of the preservative may include methyl parahydroxybenzoate, ethyl parahydroxybenzoate, sorbic acid, phenol, cresol and chlorocresol.

Besides the sulfonamide compound and Bevacizumab, the pharmaceutical composition and/or the kit of the invention can also comprise a packaging conatainer, an instruction, a package insert or the like. The packaging container, the instruction, the package insert or the like may include description of combinations for combinational use of the compound, and description of usage and dosage in the case of administering separate substances in combination or in the case of administering them in a form of a mixture. The usage and dosage may be described referring to the related description above.

The kit of the invention may also comprise: (a) at least one selected from the group consisting of a packaging container, an instruction and a package insert describing combinational use of the sulfonamide compound and Bevacizumab; and (b) a pharmaceutical composition comprising the sulfonamide compound. The kit is useful for treating cancer and/or for inhibiting angiogenesis. The pharmaceutical composition comprising the sulfonamide compound is useful for treating cancer and/or for inhibiting angiogenesis. The packaging container, the instruction, the package insert or the like may include the description of combination for combinational use of the sulfonamide compound and Bevacizumab, and description of usage and dosage for combinational use in the case of administering separate substances in combination or in the case of administering them in the form of a mixture. The usage and dosage may be described referring to the description of pharmaceutical composition and kit above.

The present invention also comprises use of a sulfonamide compound for producing a pharmaceutical composition in combination with Bevacizumab. According to the use of the invention, the pharmaceutical composition is useful for treating cancer and/or for inhibiting angiogenesis.

The present invention also comprises a method for treating cancer and/or a method for inhibiting angiogenesis comprising simultaneously or separately administering a sulfonamide compound and Bevacizumab to a patient. According to the method of the invention for treating cancer and/or the method for inhibiting angiogenesis, the route and the method for administering the sulfonamide compound and Bevacizumab are not particularly limited but reference may be made to the description of the pharmaceutical composition of the invention above.

Hereinafter, the present invention will be described by way of specific examples, although the present invention is not limited thereto.

EXAMPLE 1: Effect of Combinational use of E7820 and Bevacizumab on VEGF-induced cell proliferation in vascular endothelial cell proliferation assay *(in vitro)*

Human umbilical vein endothelial cells were suspended in Human endothelial-SFM Basal Growth Medium (Invitrogen) containing 2% FBS to 1 x $10^4$ cells/ml, and 100 μl each of this cell suspension was added to each well of a 96 well plate for cultivation in a 5% carbon dioxide incubator at 37°C. On the following day, a solution containing E7820, a solution containing Bevacizumab (Avastin® purchased from Genentech) and a solution containing both compounds, i.e., E7820 and Bevacizumab, were each diluted in a Human endothelial-SFM Basal Growth Medium containing 20 ng/ml VEGF (Genzyme Techne Corp.) and 2% FBS. These diluted solutions were added to wells under cultivation at 100 μl/well for further cultivation.

Three days later, 10 μl of cell counting kit-8 solution (Cell Counting Kit-8, Wako Pure Chemical Industries) was added, cultured for 2-3 hours at 37°C, and absorbance at 450 nm was determined with a plate reader (Corona Electric Co., Ltd.). The effect of the combinational use was calculated according to the formula of Chou et al (Adv. Enzyme Regul., 22, 27-55, 1984).

As a result, the combination of E7820 and Bevacizumab showed stronger antiproliferative effect than that obtained with E7820 or Bevacizumab alone (Table 4).

Table 4

| Concentration of compound (μg/μl) | % of control | | |
|---|---|---|---|
| | E7820 | Bevacizumab | E7820 + Bevacizumab[1)] |
| 0.0000 | 100.0 | 100.0 | 100.0 |
| 0.0006 | 98.9 | 96.0 | 97.4 |
| 0.0012 | 102.2 | 94.2 | 87.4 |
| 0.0024 | 104.8 | 89.5 | 92.3 |
| 0.0049 | 99.4 | 88.3 | 92.0 |
| 0.0098 | 97.2 | 80.8 | 82.8 |
| 0.0195 | 88.9 | 78.8 | 77.5 |
| 0.0391 | 87.9 | 71.0 | 57.4 |
| 0.0781 | 72.3 | 68.5 | 46.0 |
| 0.1563 | 60.5 | 64.2 | 38.0 |
| 0.3125 | 56.2 | 62.0 | 34.8 |
| 0.6250 | 57.2 | 60.1 | 30.5 |
| 1.2500 | 47.8 | 56.1 | 25.9 |
| 2.5000 | 41.4 | 51.8 | 23.8 |
| 5.0000 | 37.4 | 54.8 | 20.9 |
| 10.0000 | 34.4 | 53.2 | 18.7 |
| 20.0000 | 33.1 | 50.4 | 22.3 |
| 40.0000 | 12.5 | 51.7 | 12.1 |

1): "E7820 + Bevacizumab" shows the results obtained when E7820 and Bevacizumab are combined at the concentrations indicated in the left column.

Table 4 indicates percentage of absorbance of the cell treated with each compound compared to the absorbance of the untreated cell in Example 1.

Since the combination index (CI) obtained with combinational use of E7820 and Bevacizumab was 1 or less, E7820 was found to indicate a synergistic antiproliferative effect by combinational use with Bevacizumab (Table 5). In addition, CI was 0.07 or less in a fractional inhibition (fa) range as wide as 0.05-0.95, showing that synergistic effect was obtained regardless of the concentration of the compound (Table 5). This effect was remarkable as compared to an effect observed with general combinational use, which was unpredictable by those skilled in the art.

Table 5

| Fractional inhibition (fa) | Combination index (CI) | Combined effect |
|---|---|---|
| 0.05 | 0.07 | Synergistic |

(continued)

| Fractional inhibition (fa) | Combination index (CI) | Combined effect |
|---|---|---|
| 0.1 | 0.06 | Synergistic |
| 0.2 | 0.05 | Synergistic |
| 0.3 | 0.05 | Synergistic |
| 0.4 | 0.04 | Synergistic |
| 0.5 | 0.04 | Synergistic |
| 0.6 | 0.04 | Synergistic |
| 0.7 | 0.04 | Synergistic |
| 0.8 | 0.04 | Synergistic |
| 0.9 | 0.03 | Synergistic |
| 0.95 | 0.03 | Synergistic |

Table 5 shows synergistic effect of E7820 and Bevacizumab on VEGF-induced cell proliferation in a vascular endothelial cell proliferation assay *(in vitro*).

From the above results, the combination of E7820 and Bevacizumab provides a pharmaceutical composition and a kit that show a remarkable angiogenesis inhibitory activity, and a method for treating cancer and/or a method for inhibiting angiogenesis. Thus, the pharmaceutical composition and the kit of the invention can be used for treating cancer and for inhibiting angiogenesis.

EXAMPLE 2: Combinational use of E7820 and Bevacizumab in subcutaneous transplant model (*in vivo*) of human colon cancer cell line (Colo320DM)

Human colon cancer cell line Colo320DM (purchased from Dainippon Pharmaceutical) was cultured in RPMI1640 (containing 10% FBS) in a 5% carbon dioxide incubator at 37°C to about 80% confluence, and the cells were collected with trypsin-EDTA. Using a phosphate buffer containing 50% matrigel, 5 x $10^7$ cells/mL suspension was prepared, and 0.1 mL each of the resulting cell suspension was subcutaneously transplanted to a nude mouse at the side of its body. Seven days after the transplantation, administration of E7820 (200 mg/kg, twice a day, for 3 weeks, orally administered) and administration of Bevacizumab (25 mg/kg, twice a week, for 3 weeks, intravenously administered) were initiated. The major and minor axes of tumors were measured with Digimatic caliper (Mitsutoyo), and tumor volumes and relative tumor volumes were calculated according to the following formulae.

$$\text{Tumor Volume TV} = \text{Major axis of tumor (mm)} \times (\text{Minor axis of tumor})^2\ (\text{mm}^2)/2$$

$$\text{Relative Tumor Volume RTV} = \text{Tumor volume on measurement day/Tumor volume on the first administration day}$$

If statistically significant interaction was observed in the combinational use group by two-way ANOVA, a synergistic effect was considered to exist between E7820 and Bevacizumab.

As a result, E7820 was found to produce a synergistic effect when used in combination with Bevacizumab, and their combinational use showed a superior anti-tumor effect as compared with those obtained with E7820 or Bevacizumab alone (Table 6 and Figure 1). In addition, combinational use of E7820 and Bevacizumab also showed a remarkable anti-tumor effect that cannot be seen with Bevacizumab alone (Table 6 and Figure 1).

Table 6

| Administered subject | Relative tumor volume on Day 22 Average ± standard deviation | Two-way ANOVA |
|---|---|---|
| Control (untreated) | 19.0 ± 3.4 | |
| E7820 200 mg/kg | 6.1 ± 1.8 | |
| Bevacizumab 25 mg/kg | 5.1 ± 0.8 | |
| E7820 200 mg/kg + Bevacizumab 25 mg/kg | 0.8 ± 0.3 | $p < 0.01$ Synergistic effect |

Table 6 shows anti-tumor effects obtained by the use of E7820 alone, the use of Bevacizumab alone and the combinational use of E7820 and Bevacizumab in subcutaneous transplant models of human colon cancer cell line (Colo320DM). The first day of administration was considered Day 1.

From the obtained results, the combination of E7820 and Bevacizumab provides a pharmaceutical composition and a kit that show a remarkable anti-tumor activity, and a method for treating cancer, and thus the pharmaceutical composition, the kit and the method of the invention can be used for treating cancer.

EXAMPLE 3: DNA microarray analysis

(1) Cell culture, compound treatment and RNA extraction

For the purpose of examining changes in the gene expression induced by the compounds by a DNA microarray analysis, human colon cancer-derived cell line HCT116 (American Type Culture Collection, Manassas, VA, U.S.A.) and human leukemia-derived cell line MOLT-4 (American Type Culture Collection, Manassas, VA, U.S.A.) were cultured in RPMI-1640 media supplemented with 10% fetal bovine serum, 100 units/ml penicillin and 100 μg/ml streptomycin. The following cultivation and compound treatment took place in an incubator set to 5% $CO_2$ and 37°C. The HCT116 cells and the MOLT-4 cells were seeded on 10 cm-diameter cell culture dishes at 2.0 x $10^6$ cells/dish, cultured for 24 hours and subjected to the following compound treatments.

For the HCT116 cells, 12 compounds, i.e., E7820 (0.8 μM), E7070 (0.8 μM), LY295501 (30 μM), CQS (8 μM), adriamycin (0.2 μM), daunomycin (0.2 μM), ICRF154 (80 μM), ICRF159 (80 μM), kenpaullone (10 μM), alsterpullone (10 μM), trichostatin A (0.1 μM) and rapamycin (80 μM) were assessed. On the other hand, for the MOLT-4 cells, E7070 (0.8 μM) was assessed. Herein, adriamycin and daunomycin are compounds known as DNA intercalative DNA topoisomerase II inhibitors, ICRF154 and ICRF159 are compounds known as catalytic DNA topoisomerase II inhibitors, kenpaullone and alsterpullone are compounds known as cyclin-dependent kinase (CDK) inhibitors, trichostatin A is a compound known as a histone deacetylase inhibitor and rapamycin is a compound known as an mTOR/FRAP inhibitor. The concentration of each compound used for the treatment was set to three to five-fold the 50% growth inhibitory concentration of each compound to the HCT116 cells (based on three days of antiproliferative activity using WST-8). The cells were collected 24 hours after the treatment at the concentration indicated in parentheses following each compound name above. Similarly, cells cultured for 24 hours without the addition of any compound were also collected.

Extraction of total RNA from the collected cells was performed using TRIZOL reagent (Invitrogen) according to the attached instruction.

(2) Analysis of gene expression using DNA microarray

The resulting RNA was dissolved in 100 μl of diethylpyrocarbonate (DEPC)-treated sterilized water, purified using an RNeasy column (QIAGEN), and double-stranded cDNA was synthesized using SuperScript Choice System (Invitrogen) and T7-d$(T)_{24}$ primers.

First, to 10 μg RNA, 5 μM T7-d$(T)_{24}$ primer, 1x First strand buffer, 10 mM DTT, 500 μM dNTP mix and 20 units/μl SuperScript II Reverse Transcriptase were added and reacted at 42°C for an hour to synthesize single-stranded DNA. Subsequently, 1x Second strand buffer, 200 μM dNTP mix, 67 U/ml DNA ligase, 270 U/ml DNA polymerase I and 13 U/ml RNase H were added and reacted at 16°C for two hours to synthesize double-stranded cDNA. Furthermore, 67 U/ml T4 DNA polymerase I was added, reacted at 16°C for 5 minutes and then 10 μl of 0.5 M EDTA was added to terminate the reaction.

The obtained cDNA was purified with phenol/chloroform, and subjected to labeling reaction with biotinylated UTP and CTP using RNA Transcript Labeling Kit (Enzo Diagnostics) according to the attached instruction. The reaction product was purified using an RNeasy column, heated in 200 mM Tris acetic acid (pH8.1), 150 mM magnesium acetate

and 50 mM potassium acetate at 94°C for 35 minutes for fragmentation of the cRNA.

The fragmented cRNA was hybridized to GeneChip (Affymetrix) Human Focus array in 100 mM MES, 1 M sodium salt, 20 mM EDTA and 0.01% Tween 20 at 45°C for 16 hours. After the hybridization, GeneChip was washed and stained according to protocol Midi_euk2 attached to the Affymetrix fluidics station. For staining, streptavidin-phycoerythrin and biotinylated anti-streptavidin goat antibody were used. The stained GeneChip was scanned using HP confocal microscope with argon ion laser (Hewlett Packard) to determine fluorescence intensity. Measurement took place at excitation and emission wavelengths of 488 nm and 570 nm, respectively.

All of the quantitative data analyses were carried out using GeneChip software (Affymetrix) and Gene Spring (Silicongenetics). GeneChip software was used for assessing changes in the gene expression induced by each compound, where gene expression was judged to have significantly "increased" or "decreased" when the quantified values in the two conditions, i.e., between the compound-treated group and the untreated group, were twice or more as different. Gene Spring was used for assessing the similarity of changes in gene expression induced by each compound, where hierarchical cluster analysis was conducted based on changes in the expressions of all genes on the Human Focus Array.

The results from the hierarchical cluster analysis for the HCT116 cells are shown in Figure 2.

As a result of the analysis, adriamycin and daunomycin, ICRF154 and ICRF159, and Kenpaullone and alsterpullone, each pair having the same action mechanism, gave similar genetic alterations (Figure 2). Thus, compounds having the same action mechanism were confirmed to give similar genetic alterations.

E7070, E7820, LY295501 and CQS gave similar genetic alterations (Figure 2). Therefore, E7070, E7820, LY295501 and CQS were considered to have the same or similar action mechanisms according to this analysis, strongly suggesting that they give the same or similar genetic alterations and effects.

EXAMPLE 4: DNA microarray analysis

HCT116 cells were cultured in an RPMI-1640 medium supplemented with 10% fetal bovine serum, 100 units/ml penicillin and 100 μg/ml streptomycin. The following cultivation and compound treatment were carried out in an incubator at 5% $CO_2$ and 37°C. HCT116 cells were seeded in 10 cm-diameter cell culture dishes at 2.0 x $10^6$ cells/dish, cultured for 24 hours and subjected to the following compound treatment.

In this example, changes in the gene expression of HCT116 cells upon treatments with 12 compounds, i.e., E7820 (0.16 μM), E7070 (0.26 μM), LY186641 (59 μM), LY295501 (24 μM), LY-573636 (9.6 μM), CQS (4.0 μM), MST16 (100 μM), etoposide (3.6 μM), ethoxzolamide (410 μM), capsaicin (280 μM), trichostatin A (0.16 μM) and kenpaullone (7.1 μM) were examined.

MST16 is a compound known as a catalytic DNA topoisomerase II inhibitor, etoposide is a compound known as a DNA topoisomerase II inhibitor that induces formation of a cleavable complex, ethoxzolamide is a compound known as a carbonic anhydrase inhibitor, capsaicin is a compound known as a tumor-specific plasma membrane NADH oxidase inhibitor, trichostatin A is a compound known as a histone deacetylase inhibitor and kenpaullone is a compound known as a cyclin-dependent kinase (CDK) inhibitor.

The concentration of each compound used for the treatment was set to twice the 50% growth inhibitory concentration of each compound to the HCT116 cells (based on three days of antiproliferative activity using MTT). The cells were collected 24 hours after the treatment at the concentration indicated in parentheses following each compound name above. Similarly, cells cultured for 24 hours without the addition of any compound were also collected.

Total RNA extraction from the collected cells was performed using TRIZOL reagent (Invitrogen) according to the attached instruction.

Gene expression analysis using a DNA microarray was carried out in the same manner as "(2) Analysis of gene expression using DNA microarray" in Example 3.

This example was conducted for each sample in duplicate (for the convenience of the experiment, samples were given branch numbers like control-1, control-2, E7070-1, E7070-2 and so on for distinction). Then, GeneChip (Affymetrix) system (Human Focus array) was used for analyzing changes in the gene expression induced by each compound.

Twenty-six ".cel" files obtained in this example (13 samples (a control + 12 compounds) x 2) were subjected to RMA method (robust multi-array average method (Biostatistics (2003), 4, 249-264)) for normal distribution at probe level, and then the logarithm value of the signal intensity at gene level was calculated. Next, the logarithm value of the signal intensity of the untreated group (control-1) was subtracted from the logarithm value of the signal intensity of the compound-treated group for each gene to obtain the logarithm value of the signal ratio of the compound-treated group to control-1. Then, cosine correlation coefficients were calculated as correlation coefficients between the experiments (Figure 3). Based on these correlation coefficients, hierarchical cluster analysis was performed according to UPGMA method (Unweighted Pair Group Method with Arithmetic mean method) (Figure 4). Control-2 was also subjected to similar calculation (Figures 5 and 6). The softwares used were R 2.0.1 (http://www.r-project.org/) and affy package 1.5.8 (http://www.bioconductor.org).

In Figures 3-6, "LY1" represents LY186641, "LY2" represents LY295501, "LY5" represents LY573636, "CAI" represents ethoxzolamide, "Cap" represents capsaicin, "MST" represents MST16, "Etop" represents etoposide, "TSA" represents trichostatin A, and "Kenp" represents kenpaullone. In Figures 4 and 6, "de hclust (*, "average")" is a command upon statistical analysis, showing that clustering analysis is conducted by R using the average value of the duplicate experiment data.

As a result of the analysis, E7070, E7820, LY186641, LY295501, LY573636 and CQS showed very similar genetic alterations for the HCT116 cells, and were found to be different from the profiles of any of the other compounds (MST16, etoposide, ethoxzolamide, capsaicin, trichostatin A and kenpaullone) (Figures 3-6). Thus, by this analysis, E7070, E7820, LY186641, LY295501, LY573636 and CQS were considered to have the same or similar action mechanisms, strongly suggesting that they give the same or similar genetic alterations and effects.

EXAMPLE 5: Experiment on cancer cell line panels

Human cancer cell panels from 36 cell lines were used to examine correlation of antiproliferative activities among E7820, E7070, CQS, LY186641 and LY295501. The 36 types of cancer cell lines used were DLD-1, HCT15, HCT116, HT29, SW480, SW620 and WiDr (which are human colon cancer cell lines), A427, A549, LX-1, NCI-H460, NCI-H522, PC-9 and PC-10 (which are human lung cancer cell lines), GT3TKB, HGC27, MKN1, MKN7, MKN28 and MKN74 (which are human gastric cancer cell lines), AsPC-1, KP-1, KP-4, MiaPaCaII, PANC-1 and SUIT-2 (which are human pancreas cancer cell lines), BSY-1, HBC5, MCF-7, MDA-MB-231, MDA-MB-435 and MDA-MB-468 (which are human breast cancer cell lines), and CCRF-CEM, HL60, K562 and MOLT-4 (which are human leukemia cell lines). All of the cells were cultured using RPMI-1640 media supplemented with 10% fetal bovine serum, 100 units/ml penicillin and 100 μg/ml streptomycin under the conditions of 5% $CO_2$ and 37°C (Table 7).

Table 7

**36 human cancer cell lines tested in 3-day MTT assays**

| Colon | Stomach | Breast |
|---|---|---|
| DLD-1 (1250/well, 16.8 h) | GT3TKB (2000/well, 21.1 h) | BSY-1 (2000/well, 46.1 h) |
| HCT15 (1500/well, 14.5 h) | HGC27 (1500/well, 14.6 h) | HBC5 (2000/well, 31.8 h) |
| HCT116 (1250/well, 13.4 h) | MKN1 (4000/well, 35.9 h) | MCF-7 (3000/well, 29.5 h) |
| HT29 (2500/well, 19.8 h) | MKN7 (3000/well, 37.4 h) | MDA-MB231 (2000/well, 21.6 h) |
| SW480 (3000/well, 19.5 h) | MKN28 (2000/well, 22.7 h) | MDA-MB-435 (3000/well, 24.4 h) |
| SW620 (2500/well, 17.3 h) | MKN74 (4000/well, 24.8 h) | MDA-MB-468 (3000/well, 34.2 h) |
| WiDr (2000/well, 18.9 h) | | |
| **Lung** | **Pancreas** | **Leukemia** |
| A427 (2500/well, 32.4 h) | AsPC-1 (2500/well, 28.4 h) | CCRF-CEM (1500/well, 27.2 h) |
| A549 (1250/well, 18.9 h) | KP-1 (2000/well, 24.8 h) | HL60 (1500/well, 29.5 h) |
| LX-1 (2000/well, 17.2 h) | KP-4 (2000/well, 16.7 h) | K562 (1500/well, 20.6 h) |
| NCI-H460 (1000/well, 13.6 h) | MiaPaCall (2500/well, 19.1 h) | MOLT-4 (1500/well, 22.3 h) |
| NCI-H522 (4000/well, 80.4 h) | PANC-1 (2500/well, 27.9 h) | |
| PC-9 (2000/well, 23.7 h) | SUIT-2 (2000/well, 15.6 h) | |
| PC-10 (2000/well, 24.0 h) | | |
| **Cell line (initial cell number, doubling time)** | | |

Table 7 shows the types, seeded cell numbers and doubling times of the human cancer cell lines in the human cancer cell line panels.

The cells were seeded on a 96-well microplate (flat bottom) at the number indicated in Table 7 (50 μl/well). Twenty-four hours later, they were added with a 3-fold dilution series of each compound (50 μl/well). Seventy-two hours later, WST-8 (10 μl/well) was added and absorbance at 450 nm was determined. The 50% growth inhibitory concentrations to all of the 36 cancer cell lines were obtained by a least square method and their patterns were compared between the compounds. As the correlation index, Pearson's correlation coefficients were used (Paull, K. D. et al. Display and analysis of patterns of differential activity of drugs against human tumor cell lines: development of mean graph and COMPARE algorithm. J. Natl. Cancer Inst. 1989, 81, 1088-1092; Monks, A. et al. Feasibility of a high-flux anticancer drug screen using a diverse panel of cultured human tumor cell lines. J. Natl. Cancer Inst. 1991, 83, 757-766).

As a result, E7070, E7820, LY186641, LY295501 and CQS showed high correlation coefficients in antiproliferative activities against each cancer cell line (Table 8). Thus, by this analysis, E7070, E7820, LY186641, LY295501

and CQS were considered to have the same or similar action mechanisms, strongly suggesting that they give the same or similar genetic alterations and effects.

Table 8

| | E7070 | E7820 | CQS | LY186641 | LY295501 |
|---|---|---|---|---|---|
| E7070 | 1.00 | 0.98 | 0.97 | 0.93 | 0.80 |
| E7820 | 0.98 | 1.00 | 0.96 | 0.95 | 0.82 |
| CQS | 0.97 | 0.96 | 1.00 | 0.92 | 0.82 |
| LY186641 | 0.93 | 0.95 | 0.92 | 1.00 | 0.81 |
| LY295501 | 0.80 | 0.82 | 0.82 | 0.81 | 1.00 |

Table 8 shows correlation coefficients between the compounds (E7070, E7820, CQS, LY186641 and LY295501) on the human cancer cell line panels.

EXAMPLE 6: Cross-resistance in E7070-resistant cell line

An E7070-resistant cell line was used to assess the antiproliferative activities of E7820, LY186641, LY295501, LY-ASAP and CQS. HCT116-C9 was a substrain separated from human colon cancer-derived HCT116 (American Type Culture Collection, Manassas, VA, U.S.A.). This HCT116-C9 was cultured in the presence of E7070 while increasing the E7070 concentration by degrees, thereby obtaining E7070-resistant substrains HCT116-C9-C1 and HCT116-C9-C4 (Molecular Cancer Therapeutics, 2002, 1, 275-286).

Three cell lines, i.e., HCT116-C9, HCT116-C9-C1 and HCT116-C9-C4, were each seeded at 3000 cells/well onto a 96-well microplate (flat bottom) (50μl/well). Twenty-four hours later, they were added with a 3-fold dilution series of each compound (50 μl/well). Seventy-two hours later, the antiproliferative activities were assessed by MTT method (Mossmann T., J. Immunol. Methods, 1983, 65, 55-63). The 50% growth inhibitory concentrations to the cancer cells were obtained by a least square method.

As a result, the antiproliferative activity, i.e., IC50, of E7070 to HCT116-C9 (C9) was 0.127 μM. On the other hand, activities to HCT116-C9-C1 (C9C1) and HCT116-C9-C4 (C9C4) were IC50 = 31.9 μM and 26.9 μM, respectively, confirming that the antiproliferative activities of E7070 to C9C1 and C9C4 were remarkably low (Figure 7). The antiproliferative activities of E7820, CQS, LY186641, LY295501 and LY-ASAP to HCT116-C9 were IC50 = 0.080 μM, 1.73 μM, 33.6 μM, 10.9 μM and 1.63 μM, respectively while their activities to HCT116-C9-C1 were IC50 = 51.2 μM, 634 μM, 134 μM, 111 μM and 113 μM, respectively and their activities to HCT116-C9-C4 were IC50 = 52.8 μM, 517 μM, 138 μM, 110 μM and 90.3 μM, respectively. Therefore, the antiproliferative activities of E7820, CQS, LY186641, LY295501 and LY-ASAP to C9C1 and C9C4 were far lower than those to C9 (Figure 7). Thus, E7070, E7820, LY186641, LY295501, LY-ASAP and CQS were considered to have the same or similar action mechanisms, strongly suggesting that they give the same or similar genetic alterations and effects.

EXAMPLE 7: Cross-resistance in E7070-resistant cell line

In exactly the same manner as in Example 6, an E7070-resistant cell line was used to assess the antiproliferative activities of LY573636 as well as those of E7070.

As a result, the antiproliferative activities of E7070 to HCT116-C9-C1 and HCT116-C9-C4 (IC50 = 32.7 μM and 28.0 μM, respectively) were again confirmed to be remarkably lower than the activity to HCT116-C9 (IC50 = 0.127 μM) (Figure 8). The antiproliferative activities of LY573636 to HCT116-C9-C1 and HCT116-C9-C4 (IC50 = 264 μM and 240 μM, respectively) were also remarkably lower than the activity to HCT116-C9 (IC50 = 5.11 μM) (Figure 8). Thus, LY573636 was considered to have the same or similar action mechanism as that of E7070, strongly suggesting that it gives the same or similar genetic alteration and effect.

These results (Examples 3-7) confirmed that E7070, E7820, LY186641, LY295501, LY-ASAP, LY573636, CQS or a combination thereof give the same or similar genetic alterations and thus the same or similar actions and effects.

Accordingly, similar to E7820 (Examples 1, 2 and 9) or E7070 (Example 8), a sulfonamide compound, preferably E7820, E7070, LY186641, LY295501, LY-ASAP, LY573636, CQS or a combination thereof was found to show a remarkable anti-tumor activity and angiogenesis inhibitory activity upon combinational use with Bevacizumab.

EXAMPLE 8: Combinational use of E7070 and Bevacizumab in subcutaneous transplant model of human colon cancer cell line (Colo320DM)

Human colon cancer cell line Colo320DM (purchased from ATCC) was cultured in RPMI1640 (containing 10% FBS) in a 5% carbon dioxide incubator to about 80% confluence, and the cells were collected with trypsin-EDTA. Using Hanks balanced solution, 5 x $10^7$ cells/mL suspension was prepared, and 0.1 mL each of the resulting cell suspension was subcutaneously transplanted to a nude mouse at the side of its body.
Following transplantation, E7070 (40 mg/kg/day) and Bevacizumab (25 mg/kg/day) were administered alone or in combination when the average tumor volume became 259 $mm^3$.
E7070 alone was intravenously administered once a day for 5 days (on Days 1 to 5). On the other hand, Bevacizumab alone was intravenously administered twice a week for 2 weeks (on Days 1, 5, 8 and 12).
For the combinational use group, E7070 was intravenously administered on Days 1 to 5 while Bevacizumab was intravenously administered on Days 1, 5, 8 and 12.
The major and minor axes of tumors were measured twice a week from the initiation of administration with Digimatic caliper (Mitsutoyo), and tumor volumes were calculated according to the following formula.

$$\text{Tumor Volume TV} = \text{Major axis of tumor (mm)} \times (\text{Minor axis of tumor})^2\ (\text{mm}^2)/2$$

The following two values were used for judging an anti-tumor effect.

$T_{x4}$: time (days) required for the tumor to grow to four times the initial tumor vo lume
RTV: Relative tumor volume (RTV)* on Day 23
*RTV = Tumor volume on Day 23/Initial tumor volume on Day 1

When the combinational use group shows a remarkable anti-tumoral effect than those of the groups treated with E7070 or Bevacizumab alone and when statistically significant ($P<0.05$) interaction was observed by two-way ANOVA, a synergistic effect was considered to exist. When the combinational use group shows a remarkable anti-tumoral effect than those of the groups treated with E7070 or Bevacizumab alone and when $0.05 < P < 0.10$, a synergistic tendency was considered to exist.
As a result, combinational use of E7070 and Bevacizumab showed a superior anti-tumor effect as compared with the effect obtained with E7070 and Bevacizumab alone (Table 9 and Figure 9). In addition, combinational use of E7070 and Bevacizumab showed a remarkable effect that cannot be seen with Bevacizumab alone (Table 9 and Figure 9).

Table 9

| Administered subject | $T_{x4}$ | | Relative Tumor Volume (RTV) | |
|---|---|---|---|---|
| | Average ± standard deviation (days) | Two-way ANOVA | Average ± standard deviation | Two-way ANOVA |
| Control (untreated) | 9.3 ± 1.3 | | 12.23 ± 1.80 | |
| E7070 40 mg/kg | 27.0 ± 3.4 | | 2.70 ± 0.97 | |
| Bevacizumab 25 mg/kg | 26.3 ± 2.8 | | 2.84 ± 0.68 | |
| E7070 40 mg/kg + Bevacizumab 25 mg/kg (Combinational use) | 52.4 ± 6.8 | P = 0.057 Synergistic tendency | 1.07 ± 0.29 | P = 0.060 Synergistic tendency |

Table 9 shows anti-tumor effects obtained by the use of E7070 alone, the use of Bevacizumab alone and the combinational use of E7070 and Bevacizumab in subcutaneous transplant models of human colon cancer cell line (Colo320DM). The first day of administration was considered Day 1.
From the above results, the combination of E7070 and Bevacizumab was confirmed to provide a pharmaceutical

composition and a kit that show a remarkable anti-tumoral activity and a method for treating cancer, and thus the pharmaceutical composition, the kit and the method of the invention can be used for treating cancer.

EXAMPLE 9: Combinational use of E7820 and Bevacizumab in subcutaneous transplant model of human renal cancer cell line (786-O)

Human renal cancer cell line 786-O (obtained from ATCC) was cultured in RPMI1640 (containing 10% FBS) in a 5% carbon dioxide incubator at 37°C to about 80% confluence, and the cells were collected with trypsin-EDTA. Using a phosphate buffer containing 50% matrigel, 1 x $10^8$ cells/mL suspension was prepared, and 0.1 mL each of the resulting cell suspension was subcutaneously transplanted to a nude mouse at the side of its body.

Seven days after the transplantation, E7820 and Bevacizumab were administered alone or in combination. E7820 was orally administered at 200 mg/kg twice a day for 2 weeks while Bevacizumab was intravenously administered at 25 mg/kg twice a week for 2 weeks.

The major and minor axes of tumors were measured with Digimatic caliper (Mitsutoyo), and tumor volumes and relative tumor volumes were calculated according to the following formulae.

$$\text{Tumor Volume TV} = \text{Major axis of tumor (mm)} \times (\text{Minor axis of tumor})^2\ (\text{mm}^2)/2$$

$$\text{Relative Tumor Volume RTV} = \text{Tumor volume on measurement day/Tumor volume on the first administration day}$$

When statistically significant interaction was observed in the combinational use group by two-way ANOVA, a synergistic effect was considered to exist.

As a result, combinational use of E7820 and Bevacizumab showed a synergistic effect and a superior anti-tumor effect as compared with the effect obtained with E7820 or Bevacizumab alone (Table 10). Moreover, combinational use of E7820 and Bevacizumab showed a remarkable anti-tumor effect that cannot be seen with Bevacizumab alone (Table 10).

Table 10

| Administered drug | Relative tumor volume on Day 22 Average ± standard deviation | Two-way ANOVA |
|---|---|---|
| Control (untreated) | 1.6 ± 0.2 | |
| E7820 200 mg/kg | 0.6 ± 0.1 | |
| Bevacizumab 25 mg/kg | 1.8 ± 0.2 | |
| E7820 200 mg/kg + Bevacizumab 25 mg/kg | 0.4 ± 0.1 | $p < 0.05$ synergistic effect |

Table 10 shows anti-tumor effects obtained by the use of E7820 alone, the use of Bevacizumab alone and the combinational use of E7820 and Bevacizumab in subcutaneous transplant models of human renal cancer cell line (786-O). The first day of administration was considered Day 1.

From the obtained results, the combinational use of E7820 and Bevacizumab was confirmed to provide a pharmaceutical composition and a kit that show a remarkable anti-tumor activity and a method for treating cancer, and thus the pharmaceutical composition, the kit and the method of the invention can be used for treating cancer.

INDUSTRIAL APPLICABILITY

The present invention provides a pharmaceutical composition and a kit that show a remarkable anti-tumor activity and/or angiogenesis inhibitory activity, and a method for treating cancer and/or a method for inhibiting angiogenesis.

More specifically, the present invention provides a pharmaceutical composition and a kit that show a remarkable

anti-tumor activity and/or angiogenesis inhibitory activity, and a method for treating cancer and/or a method for inhibiting angiogenesis, characterized by comprising a sulfonamide compound (i.e., at least one compound selected from: (A) a compound represented by General Formula (I), preferably E7070 or E7820; (B) a compound represented by General Formula (II), preferably LY186641 or LY295501; (C) a compound represented by General Formula (III), preferably LY-ASAP; (D) LY573636; and (E) CQS) in combination with Bevacizumab. The pharmaceutical composition, the kit and the method of the invention are useful for treating cancer or for inhibiting angiogenesis.

## Claims

**1.** A pharmaceutical composition comprising a sulfonamide compound in combination with Bevacizumab, wherein the sulfonamide compound is at least one compound selected from the group consisting of:

a compound represented by General Formula (I)

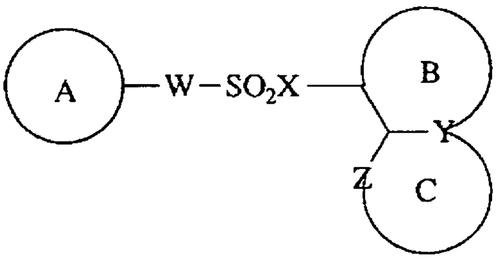

(I)

[wherein, ring A represents an optionally substituted monocyclic or bicyclic aromatic ring,
ring B represents an optionally substituted 6-membered cyclic unsaturated hydrocarbon or 6-membered unsaturated heterocycle containing a nitrogen atom as a heteroatom,
ring C represents an optionally substituted 5-membered heterocycle containing one or two nitrogen atoms,
W represents a single bond or -CH=CH-,
X represents $-N(R^1)-$ or an oxygen atom,
Y represents

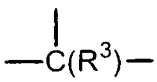 or 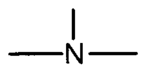 ,

Z represents $-N(R^2)-$,
wherein, $R^1$, $R^2$ and $R^3$ independently represent, identically or differently, a hydrogen atom or a lower alkyl group];
a compound represented by General Formula (II)

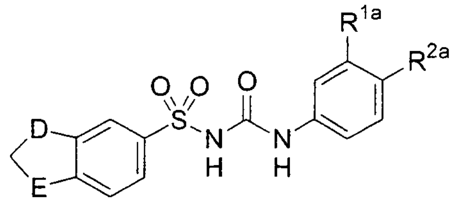

(II)

[wherein, E represents -O-, $-N(CH_3)-$, $-CH_2-$, $-CH_2CH_2-$ or $-CH_2O-$, D represents $-CH_2-$ or -O-, $R^{1a}$ represents a hydrogen atom or a halogen atom, and $R^{2a}$ represents a halogen atom or a trifluoromethyl group];
a compound represented by General Formula (III)

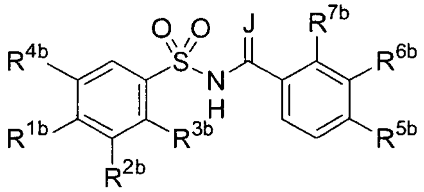
(III)

[wherein, J represents -O- or -NH-, $R^{1b}$ represents a hydrogen atom, -a halogen atom, an optionally substituted $C_1$-$C_6$ alkyl group, an optionally substituted $C_1$-$C_4$ alkoxy group, an optionally substituted $C_1$-$C_4$ alkylthio group, $-CF_3$, $-OCF_3$, $-SCF_3$, an optionally substituted $C_1$-$C_4$ alkoxy carbonyl group, a nitro group, an azido group, -O $(SO_2)CH_3$, $-N(CH_3)_2$, a hydroxyl group, a phenyl group, a substituted phenyl group, a pyridinyl group, a thienyl group, a furyl group, a quinolinyl group or a triazole group, $R^{2b}$ represents a hydrogen atom, a halogen atom, a cyano group, $-CF_3$, an optionally substituted $C_1$-$C_6$ alkyl group, an optionally substituted $C_1$-$C_4$ alkoxy carbonyl group, an optionally substituted $C_1$-$C_4$ alkoxy group, an optionally substituted phenyl group or an optionally substituted quinolinyl group, $R^{3b}$ represents a hydrogen atom or an optionally substituted $C_1$-$C_4$ alkoxy group, $R^{4b}$ represents a hydrogen atom or an optionally substituted $C_1$-$C_6$ alkyl group (provided that at least one of $R^{3b}$ and $R^{4b}$ is a hydrogen atom), $R^{5b}$ represents a hydrogen atom, a halogen atom, an optionally substituted $C_1$-$C_6$ alkyl group, $-CF_3$ or a nitro group, $R^{6b}$ represents a hydrogen atom, a halogen atom or an optionally substituted $C_1$-$C_6$ alkyl group (provided that when $R^{6b}$ is an optionally substituted $C_1$-$C_6$ alkyl group, $R^{5b}$ is a hydrogen atom and $R^{7b}$ is a halogen atom), $R^{7b}$ represents a halogen atom, an optionally substituted $C_1$-$C_6$ alkyl group or $-CF_3$ (provided that when either $R^{5b}$ or $R^{7b}$ is an optionally substituted $C_1$-$C_6$ alkyl group or when $R^{7b}$ is a halogen atom or an optionally substituted $C_1$-$C_6$ alkyl group, either $R^{5b}$ or $R^{6b}$ is a hydrogen atom)];

a compound represented by Formula (IV)

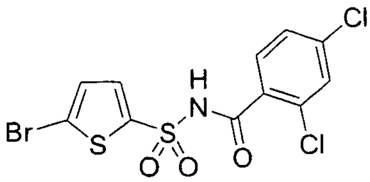
(IV)

;

and

a compound represented by Formula (V)

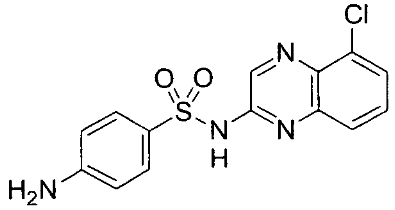
(V)

,

or a pharmacologically acceptable salt thereof, or a solvate thereof.

**2.** The pharmaceutical composition according to Claim 1, wherein the sulfonamide compound is at least one compound selected from the group consisting of N-(3-chloro-1H-indole-7-yl)-4-sulfamoylbenzenesulfonamide, N-(3-cyano-4-methyl- H-indole-7-yl)-3-cyanobenzenesulfonamide, N-[[(4-chlorophenyl)amino]carbonyl]-2,3-dihydro-1H-indene-5-sulfonamide, N-[[(3,4-dichlorophenyl)amino]carbonyl]-2,3-dihydrobenzofuran-5-sulfonamide, N-(2,4-dichlorobenzoyl)-4-chlorophenylsulfonamide, N-(2,4-dichlorobenzoyl)-5-bromothiophene-2-sulfonamide and 2-sulfanylamide-5-chloroquinoxaline, or a pharmacologically acceptable salt thereof or a solvate thereof.

**3.** The pharmaceutical composition according to Claim 1, wherein the sulfonamide compound is N-(3-cyano-4-methyl-1H-indole-7-yl)-3-cyanobenzenesulfonamide, a pharmacologically acceptable salt thereof, or a solvate thereof.

**4.** The pharmaceutical composition according to Claim 1, wherein the sulfonamide compound is N-(3-chloro-1H-indole-7-yl)-4-sulfamoylbenzenesulfonamide, a pharmacologically acceptable salt thereof, or a solvate thereof.

**5.** The pharmaceutical composition according to Claim 1, wherein the sulfonamide compound is at least one compound selected from the group consisting of N-[[(3,4-dichlorophenyl)amino]carbonyl]-2,3-dihydrobenzofuran-5-sulfonamide and N-(2,4-dichlorobenzoyl)-5-bromothiophene-2-sulfonamide, or a pharmacologically acceptable salt thereof or a solvate thereof.

**6.** The pharmaceutical composition according to Claim 1, wherein the sulfonamide compound is sodium salt of N-(2,4-dichlorobenzoyl)-5-bromothiophene-2-sulfonamide.

**7.** The pharmaceutical composition according to any one of Claims 1-6, wherein the pharmaceutical composition is used for treating cancer.

**8.** The pharmaceutical composition according to any one of Claims 1-6, wherein the pharmaceutical composition is used for inhibiting angiogenesis.

**9.** A kit comprising

(a) at least one selected from the group consisting of a packaging container, an instruction and a package insert describing the combinational use of a sulfonamide compound and Bevacizumab and
(b) a pharmaceutical composition comprising the sulfonamide compound,

wherein the sulfonamide compound is at least one compound selected from the group consisting of:

a compound represented by General Formula (I)

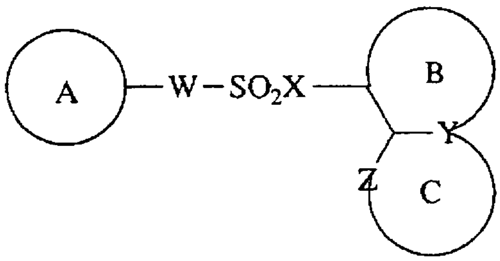

(I)

[wherein, ring A represents an optionally substituted monocyclic or bicyclic aromatic ring,
ring B represents an optionally substituted 6-membered cyclic unsaturated hydrocarbon or 6-membered unsaturated heterocycle containing a nitrogen atom as a heteroatom,
ring C represents an optionally substituted 5-membered heterocycle containing one or two nitrogen atoms,
W represents a single bond or -CH=CH-,
X represents -N($R^1$)- or an oxygen atom,
Y represents

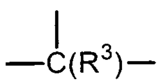 or 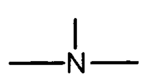 ,

Z represents -N($R^2$)-,
wherein, $R^1$, $R^2$ and $R^3$ independently represent, identically or differently, a hydrogen atom or a lower alkyl group];
a compound represented by General Formula (II)

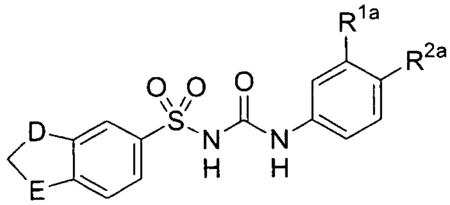

(II)

[wherein, E represents -O-, $-N(CH_3)-$, $-CH_2-$, $-CH_2CH_2-$ or $-CH_2O-$, D represents $-CH_2-$ or -O-, $R^{1a}$ represents a hydrogen atom or a halogen atom, and $R^{2a}$ represents a halogen atom or a trifluoromethyl group];
a compound represented by General Formula (III)

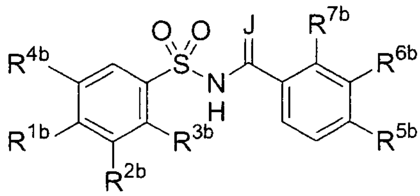

(III)

[wherein, J represents -O- or -NH-, $R^{1b}$ represents a hydrogen atom, a halogen atom, an optionally substituted $C_1$-$C_6$ alkyl group, an optionally substituted $C_1$-$C_4$ alkoxy group, an optionally substituted $C_1$-$C_4$ alkylthio group, $-CF_3$, $-OCF_3$, $-SCF_3$, an optionally substituted $C_1$-$C_4$ alkoxy carbonyl group, a nitro group, an azido group, $-O(SO_2)CH_3$, $-N(CH_3)_2$, a hydroxyl group, a phenyl group, a substituted phenyl group, a pyridinyl group, a thienyl group, a furyl group, a quinolinyl group or a triazole group, $R^{2b}$ represents a hydrogen atom, a halogen atom, a cyano group, $-CF_3$, an optionally substituted $C_1$-$C_6$ alkyl group, an optionally substituted $C_1$-$C_4$ alkoxy carbonyl group, an optionally substituted $C_1$-$C_4$ alkoxy group, an optionally substituted phenyl group or an optionally substituted quinolinyl group, $R^{3b}$ represents a hydrogen atom or an optionally substituted $C_1$-$C_4$ alkoxy group, $R^{4b}$ represents a hydrogen atom or an optionally substituted $C_1$-$C_6$ alkyl group (provided that at least one of $R^{3b}$ and $R^{4b}$ is a hydrogen atom), $R^{5b}$ represents a hydrogen atom, a halogen atom, an optionally substituted $C_1$-$C_6$ alkyl group, $-CF_3$ or a nitro group, $R^{6b}$ represents a hydrogen atom, a halogen atom or an optionally substituted $C_1$-$C_6$ alkyl group (provided that when $R^{6b}$ is an optionally substituted $C_1$-$C_6$ alkyl group, $R^{5b}$ is a hydrogen atom and $R^{7b}$ is a halogen atom), $R^{7b}$ represents a halogen atom, an optionally substituted $C_1$-$C_6$ alkyl group or $-CF_3$ (provided that when either $R^{5b}$ or $R^{7b}$ is an optionally substituted $C_1$-$C_6$ alkyl group or when $R^{7b}$ is a halogen atom or an optionally substituted $C_1$-$C_6$ alkyl group, either $R^{5b}$ or $R^{6b}$ is a hydrogen atom)];
a compound represented by Formula (IV)

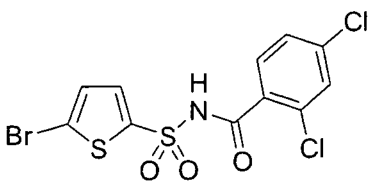

(IV)

;

and
a compound represented by Formula (V)

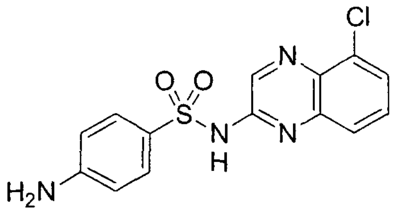 (V)

,

or a pharmacologically acceptable salt thereof, or a solvate thereof.

**10.** The kit according to Claim 9, wherein the sulfonamide compound is at least one compound selected from the group consisting of N-(3-chloro-1H-indole-7-yl)-4-sulfamoylbenzenesulfonamide, N-(3-cyano-4-methyl-1H-indole-7-yl)-3-cyanobenzenesulfonamide, N-[[(4-chlorophenyl)amino]carbonyl]-2,3-dihydro-1H-indene-5-sulfonamide, N-[[(3,4-dichlorophenyl)amino]carbonyl]-2,3-dihydrobenzofuran-5-sulfonamide, N-(2,4-dichlorobenzoyl)-4-chlorophenylsulfonamide, N-(2,4-dichlorobenzoyl)-5-bromothiophene-2-sulfonamide and 2-sulfanylamide-5-chloroqumoxalme, or a pharmacologically acceptable salt thereof, or a solvate thereof.

**11.** The kit according to Claim 9, wherein the sulfonamide compound is N-(3-cyano-4-methyl-1H-indole-7-yl)-3-cyanobenzenesulfonamide, a pharmacologically acceptable salt thereof, or a solvate thereof

**12.** The kit according to Claim 9, wherein the sulfonamide compound is N-(3-chloro-1H-indole-7-yl)-4-sulfamoylbenzenesulfonamide, a pharmacologically acceptable salt thereof, or a solvate thereof.

**13.** The kit according to Claim 9, wherein the sulfonamide compound is at least one compound selected from the group consisting of N-[[(3,4-dichlorophenyl)amino]carbonyl]-2,3-dihydrobenzofuran-5-sulfonamide and N-(2,4-dichlorobenzoyl)-5-bromothiophene-2-sulfonamide, or a pharmacologically acceptable salt thereof or a solvate thereof.

**14.** The kit according to Claim 9, wherein the sulfonamide compound is sodium salt of N-(2,4-dichlorobenzoyl)-5-bromothiophene-2-sulfonamide.

**15.** The kit according to any one of Claims 9-14, wherein the kit is used for treating cancer.

**16.** The kit according to any one of Claims 9-14, wherein the kit is used for inhibiting angiogenesis.

**17.** A kit comprising a set of a formulation comprising a sulfonamide compound and a formulation comprising Bevacizumab, wherein the sulfonamide compound is at least one compound selected from the group consisting of:

a compound represented by General Formula (I)

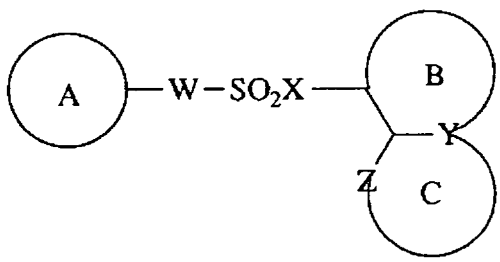 (I)

[wherein, ring A represents an optionally substituted monocyclic or bicyclic aromatic ring,
ring B represents an optionally substituted 6-membered cyclic unsaturated hydrocarbon or 6-membered unsaturated heterocycle containing a nitrogen atom as a heteroatom,
ring C represents an optionally substituted 5-membered heterocycle containing one or two nitrogen atoms,
W represents a single bond or -CH=CH-,
X represents $-N(R^1)-$ or an oxygen atom,
Y represents

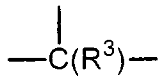 or 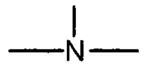 ,

Z represents $-N(R^2)-$,
wherein, $R^1$, $R^2$ and $R^3$ independently represent, identically or differently, a hydrogen atom or a lower alkyl group];
a compound represented by General Formula (II)

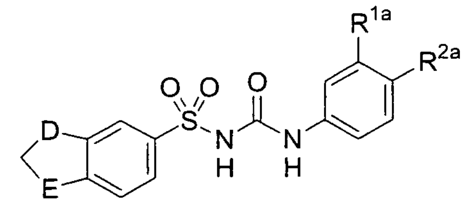 (II)

[wherein, E represents -O-, $-N(CH_3)-$, $-CH_2-$, $-CH_2CH_2-$ or $-CH_2O-$, D represents $-CH_2-$ or -O-, $R^{1a}$ represents a hydrogen atom or a halogen atom, and $R^{2a}$ represents a halogen atom or a trifluoromethyl group];
a compound represented by General Formula (III)

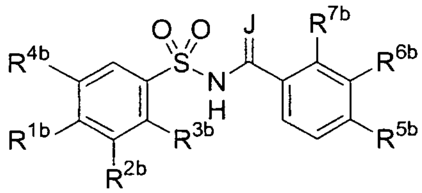 (III)

[wherein, J represents -O- or -NH-, $R^{1b}$ represents a hydrogen atom, a halogen atom, an optionally substituted $C_1$-$C_6$ alkyl group, an optionally substituted $C_1$-$C_4$ alkoxy group, an optionally substituted $C_1$-$C_4$ alkylthio group, $-CF_3$, $-OCF_3$, $-SCF_3$, an optionally substituted $C_1$-$C_4$ alkoxy carbonyl group, a nitro group, an azido group, $-O(SO_2)CH_3$, $-N(CH_3)_2$, a hydroxyl group, a phenyl group, a substituted phenyl group, a pyridinyl group, a thienyl group, a furyl group, a quinolinyl group or a triazole group, $R^{2b}$ represents a hydrogen atom, a halogen atom, a cyano group, $-CF_3$, an optionally substituted $C_1$-$C_6$ alkyl group, an optionally substituted $C_1$-$C_4$ alkoxy carbonyl group, an optionally substituted $C_1$-$C_4$ alkoxy group, an optionally substituted phenyl group or an optionally substituted quinolinyl group, $R^{3b}$ represents a hydrogen atom or an optionally substituted $C_1$-$C_4$ alkoxy group, $R^{4b}$ represents a hydrogen atom or an optionally substituted $C_1$-$C_6$ alkyl group (provided that at least one of $R^{3b}$ and $R^{4b}$ is a hydrogen atom), $R^{5b}$ represents a hydrogen atom, a halogen atom, an optionally substituted $C_1$-$C_6$ alkyl group, $-CF_3$ or a nitro group, $R^{6b}$ represents a hydrogen atom, a halogen atom or an optionally substituted $C_1$-$C_6$ alkyl group (provided that when $R^{6b}$ is an optionally substituted $C_1$-$C_6$ alkyl group, $R^{5b}$ is a hydrogen atom and $R^{7b}$ is a halogen atom), $R^{7b}$ represents a halogen atom, an optionally substituted $C_1$-$C_6$ alkyl group or $-CF_3$ (provided that when either $R^{5b}$ or $R^{7b}$ is an optionally substituted $C_1$-$C_6$ alkyl group or when $R^{7b}$ is a halogen atom or an optionally substituted $C_1$-$C_6$ alkyl group, either $R^{5b}$ or $R^{6b}$ is a hydrogen atom)];
a compound represented by Formula (IV)

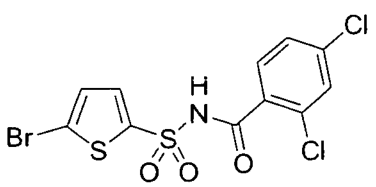 (IV)

;

and
a compound represented by Formula (V)

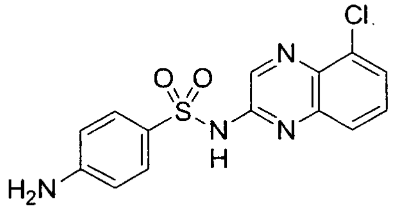

(V)

,

or a pharmacologically acceptable salt thereof or a solvate thereof.

**18.** The kit according to Claim 17, wherein the sulfonamide compound is at least one compound selected from the group consisting of N-(3-chloro-1H-indole-7-yl)-4-sulfamoylbenzenesulfonamide, N-(3-cyano-4-methyl-1H-indole-7-yl)-3-cyanobenzenesulfonamide, N-[[(4-chlorophenyl)amino]carbonyl]-2,3-dihydro-1H-indene-5-sulfonamide, N-[[(3,4-dichlorophenyl)amino]carbonyl]-2,3-dihydrobenzofuran-5-sulfonamide, N-(2,4-dichlorobenzoyl)-4-chlorophenylsulfonamide, N-(2,4-dichlorobenzoyl)-5-bromothiophene-2-sulfonamide and 2-sulfanylamide-5-chloroquinoxaline, or a pharmacologically acceptable salt thereof or a solvate thereof.

**19.** The kit according to Claim 17, wherein the sulfonamide compound is N-(3-cyano-4-methyl-1H-indole-7-yl)-3-cyanobenzenesulfonamide, a pharmacologically acceptable salt thereof, or a solvate thereof

**20.** The kit according to Claim 17, wherein the sulfonamide compound is N-(3-chloro-1H-indole-7-yl)-4-sulfamoylbenzenesulfonamide, a pharmacologically acceptable salt thereof or a solvate thereof.

**21.** The kit according to Claim 17, wherein the sulfonamide compound is at least one compound selected from the group consisting of N-[[(3,4-dichlorophenyl)amino]carbonyl]-2,3-dihydrobenzofuran-5-sulfonamide and N-(2,4-dichlorobenzoyl)-5-bromothiophene-2-sulfonamide, or a pharmacologically acceptable salt thereof, or a solvate thereof

**22.** The kit according to Claim 17, wherein the sulfonamide compound is sodium salt of N-(2,4-dichlorobenzoyl)-5-bromothiophene-2-sulfonamide.

**23.** The kit according to any one of Claims 17-22, wherein the kit is used for treating cancer.

**24.** The kit according to any one of Claims 17-22, wherein the kit is used for inhibiting angiogenesis.

**25.** Use of a sulfonamide compound for producing a pharmaceutical composition in combination with Bevacizumab, wherein the sulfonamide compound is at least one compound selected from the group consisting of:

a compound represented by General Formula (I)

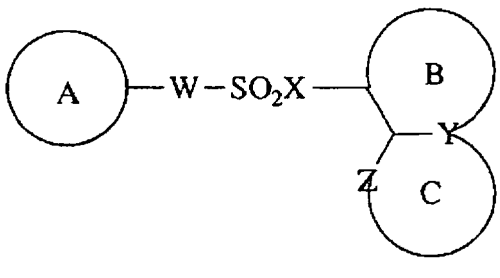

(I)

[wherein, ring A represents an optionally substituted monocyclic or bicyclic aromatic ring,
ring B represents an optionally substituted 6-membered cyclic unsaturated hydrocarbon or 6-membered unsaturated heterocycle containing a nitrogen atom as a heteroatom,
ring C represents an optionally substituted 5-membered heterocycle containing one or two nitrogen atoms,

W represents a single bond or -CH=CH-,
X represents $-N(R^1)-$ or an oxygen atom,
Y represents

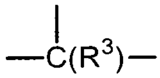
or
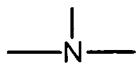
,

Z represents $-N(R^2)-$,
wherein, $R^1$, $R^2$ and $R^3$ independently represent, identically or differently, a hydrogen atom or a lower alkyl group];
a compound represented by General Formula (II)

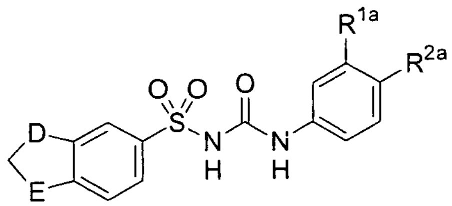
(II)

[wherein, E represents -O-, $-N(CH_3)-$, $-CH_2-$, $-CH_2CH_2-$ or $-CH_2O-$, D represents $-CH_2-$ or -O-, $R^{1a}$ represents a hydrogen atom or a halogen atom, and $R^{2a}$ represents a halogen atom or a trifluoromethyl group];
a compound represented by General Formula (III)

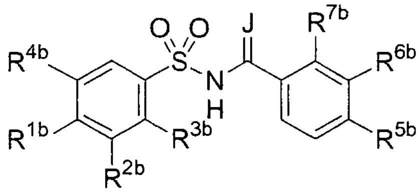
(III)

[wherein, J represents -O- or -NH-, $R^{1b}$ represents a hydrogen atom, a halogen atom, an optionally substituted $C_1$-$C_6$ alkyl group, an optionally substituted $C_1$-$C_4$ alkoxy group, an optionally substituted $C_1$-$C_4$ alkylthio group, $-CF_3$, $-OCF_3$, $-SCF_3$, an optionally substituted $C_1$-$C_4$ alkoxy carbonyl group, a nitro group, an azido group, $-O(SO_2)CH_3$, $-N(CH_3)_2$, a hydroxyl group, a phenyl group, a substituted phenyl group, a pyridinyl group, a thienyl group, a furyl group, a quinolinyl group or a triazole group, $R^{2b}$ represents a hydrogen atom, a halogen atom, a cyano group, $-CF_3$, an optionally substituted $C_1$-$C_6$ alkyl group, an optionally substituted $C_1$-$C_4$ alkoxy carbonyl group, an optionally substituted $C_1$-$C_4$ alkoxy group, an optionally substituted phenyl group or an optionally substituted quinolinyl group, $R^{3b}$ represents a hydrogen atom or an optionally substituted $C_1$-$C_4$ alkoxy group, $R^{4b}$ represents a hydrogen atom or an optionally substituted $C_1$-$C_6$ alkyl group (provided that at least one of $R^{3b}$ and $R^{4b}$ is a hydrogen atom), $R^{5b}$ represents a hydrogen atom, a halogen atom, an optionally substituted $C_1$-$C_6$ alkyl group, $-CF_3$ or a nitro group, $R^{6b}$ represents a hydrogen atom, a halogen atom or an optionally substituted $C_1$-$C_6$ alkyl group (provided that when $R^{6b}$ is an optionally substituted $C_1$-$C_6$ alkyl group, $R^{5b}$ is a hydrogen atom and $R^{7b}$ is a halogen atom), $R^{7b}$ represents a halogen atom, an optionally substituted $C_1$-$C_6$ alkyl group or $-CF_3$ (provided that when either $R^{5b}$ or $R^{7b}$ is an optionally substituted $C_1$-$C_6$ alkyl group or when $R^{7b}$ is a halogen atom or an optionally substituted $C_1$-$C_6$ alkyl group, either $R^{5b}$ or $R^{6b}$ is a hydrogen atom)];
a compound represented by Formula (IV)

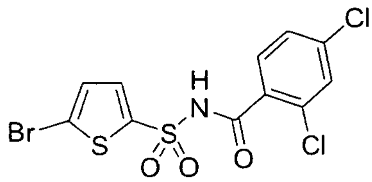

(IV)

;

and

a compound represented by Formula (V)

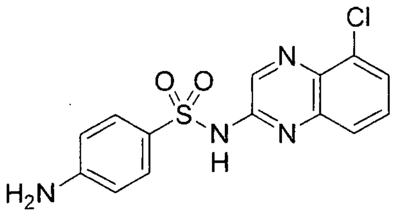

(V)

,

or a pharmacologically acceptable salt thereof or a solvate thereof.

**26.** The use according to Claim 25, wherein the sulfonamide compound is at least one compound selected from the group consisting of: N-(3-chloro-1H-indole-7-yl)-4-sulfamoylbenzenesulfonamide; N-(3-cyano-4-methyl-1H-indole-7-yl)-3-cyanobenzenesulfonamide; N-[[(4-chlorophenyl)amino]carbonyl]-2,3-dihydro-1H-indene-5-sulfonamide; N-[[(3,4-dichlorophenyl)amino]carbonyl]-2,3-dihydrobenzofuran-5-sulfonamide; N-(2,4-dichlorobenzoyl)-4-chlorophenylsulfonamide; N-(2,4-dichlorobenzoyl)-5-bromothiophene-2-sulfonamide; and 2-sulfanylamide-5-chloroquinoxaline, or a pharmacologically acceptable salt thereof or a solvate thereof.

**27.** The use according to Claim 25, wherein the sulfonamide compound is N-(3-cyano-4-methyl-1H-indole-7-yl)-3-cyanobenzenesulfonamide, a pharmacologically acceptable salt thereof, or a solvate thereof

**28.** The use according to Claim 25, wherein the sulfonamide compound is N-(3-chloro-1H-indole-7-yl)-4-sulfamoylbenzenesulfonamide, a pharmacologically acceptable salt thereof, or a solvate thereof.

**29.** The use according to Claim 25, wherein the sulfonamide compound is at least one compound selected from the group consisting of N-[[(3,4-dichlorophenyl)amino]carbonyl]-2,3-dihydrobenzofuran-5-sulfonamide and N-(2,4-dichlorobenzoyl)-5-bromothiophene-2-sulfonamide, or a pharmacologically acceptable salt thereof or a solvate thereof.

**30.** The use according to Claim 25, wherein the sulfonamide compound is sodium salt of N-(2,4-dichlorobenzoyl)-5-bromothiophene-2-sulfonamide.

**31.** The use according to any one of Claims 25-30, wherein the pharmaceutical composition is used for treating cancer.

**32.** The use according to any one of Claims 25-30, wherein the pharmaceutical composition is used for inhibiting angiogenesis.

**33.** A method for treating cancer and/or a method for inhibiting angiogenesis, comprising administering a sulfonamide compound and Bevacizumab to a patient, wherein the sulfonamide compound is at least one compound selected from the group consisting of:

a compound represented by General Formula (I)

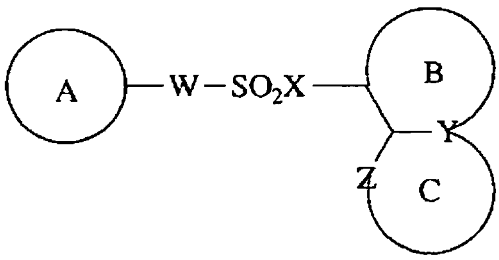

(I)

[wherein, ring A represents an optionally substituted monocyclic or bicyclic aromatic ring,
ring B represents an optionally substituted 6-membered cyclic unsaturated hydrocarbon or 6-membered unsaturated heterocycle containing a nitrogen atom as a heteroatom,
ring C represents an optionally substituted 5-membered heterocycle containing one or two nitrogen atoms,
W represents a single bond or -CH=CH-,
X represents $-N(R^1)-$ or an oxygen atom,
Y represents

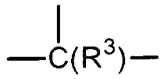 or 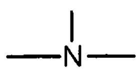 ,

Z represents $-N(R^2)-$,
wherein, $R^1$, $R^2$ and $R^3$ independently represent, identically or differently, a hydrogen atom or a lower alkyl group];
a compound represented by General Formula (II)

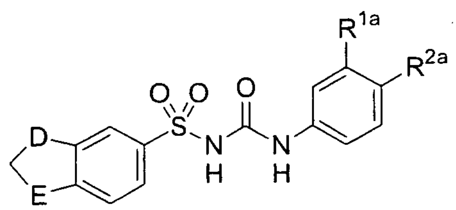

(II)

[wherein, E represents -O-, $-N(CH_3)-$, $-CH_2-$, $-CH_2CH_2-$ or $-CH_2O-$, D represents $-CH_2-$ or -O-, $R^{1a}$ represents a hydrogen atom or a halogen atom, and $R^{2a}$ represents a halogen atom or a trifluoromethyl group];
a compound represented by General Formula (III)

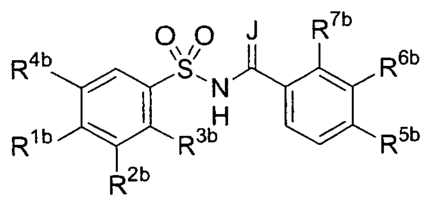

(III)

[wherein, J represents -O- or -NH-, $R^{1b}$ represents a hydrogen atom, a halogen atom, an optionally substituted $C_1$-$C_6$ alkyl group, an optionally substituted $C_1$-$C_4$ alkoxy group, an optionally substituted $C_1$-$C_4$ alkylthio group, $-CF_3$, $-OCF_3$, $-SCF_3$, an optionally substituted $C_1$-$C_4$ alkoxy carbonyl group, a nitro group, an azido group, $-O(SO_2)CH_3$, $-N(CH_3)_2$, a hydroxyl group, a phenyl group, a substituted phenyl group, a pyridinyl group, a thienyl group, a furyl group, a quinolinyl group or a triazole group, $R^{2b}$ represents a hydrogen atom, a halogen atom, a cyano group, $-CF_3$, an optionally substituted $C_1$-$C_6$ alkyl group, an optionally substituted $C_1$-$C_4$ alkoxy carbonyl group, an optionally substituted $C_1$-$C_4$ alkoxy group, an optionally substituted phenyl group or an optionally substituted quinolinyl group, $R^{3b}$ represents a hydrogen atom or an optionally substituted $C_1$-$C_4$ alkoxy group, $R^{4b}$ represents a hydrogen atom or an optionally substituted $C_1$-$C_6$ alkyl group (provided that at least one of $R^{3b}$ and $R^{4b}$ is a hydrogen atom), $R^{5b}$ represents a hydrogen atom, a halogen atom, an optionally substituted $C_1$-$C_6$ alkyl group, $-CF_3$ or a nitro group, $R^{6b}$ represents a hydrogen atom, a halogen atom or an optionally substituted $C_1$-$C_6$ alkyl group (provided that when $R^{6b}$ is an optionally substituted $C_1$-$C_6$ alkyl group, $R^{5b}$ is a hydrogen atom and $R^{7b}$ is a halogen atom), $R^{7b}$ represents a halogen atom, an optionally substituted $C_1$-$C_6$ alkyl group or $-CF_3$ (provided that when either $R^{5b}$ or $R^{7b}$ is an optionally substituted $C_1$-$C_6$ alkyl group or when $R^{7b}$ is a halogen atom or an optionally substituted $C_1$-$C_6$ alkyl group, either $R^{5b}$ or $R^{6b}$ is a hydrogen atom)];

a compound represented by Formula (IV)

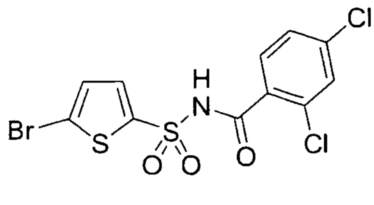

(IV)

;

and

a compound represented by Formula (V)

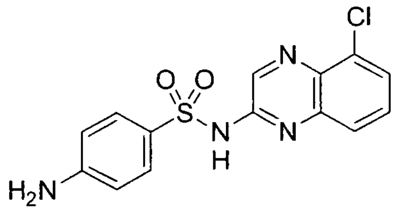

(V)

,

or a pharmacologically acceptable salt thereof or a solvate thereof.

**34.** The method according to Claim 33, wherein the sulfonamide compound is at least one compound selected from the group consisting of: N-(3-chloro-1H-indole-7-yl)-4-sulfamoylbenzenesulfonamide; N-(3-cyano-4-methyl-1H-indole-7-yl)-3-cyanobenzenesulfonamide; N-[[(4-chlorophenyl)amino]carbonyl]-2,3-dihydro-1H-indene-5-sulfonamide; N-[[(3,4-dichlorophenyl)amino]carbonyl]-2,3-dihydrobenzofuran-5-sulfonamide; N-(2,4-dichlorobenzoyl)-4-chlorophenylsulfonamide; N-(2,4-dichlorobenzoyl)-5-bromothiophene-2-sulfonamide; and 2-sulfanylamide-5-chloroquinoxaline, or a pharmacologically acceptable salt thereof or a solvate thereof.

**35.** The method according to Claim 33, wherein the sulfonamide compound is N-(3-cyano-4-methyl-1H-indole-7-yl)-3-cyanobenzenesulfonamide, a pharmacologically acceptable salt thereof or a solvate thereof.

**36.** The method according to Claim 33, wherein the sulfonamide compound is N-(3-chloro-1H-indole-7-yl)-4-sulfamoylbenzenesulfonamide, a pharmacologically acceptable salt thereof or a solvate thereof.

**37.** The method according to Claim 33, wherein the sulfonamide compound is at least one compound selected from the group consisting of N-[[(3,4-dichlorophenyl)amino]carbonyl]-2,3-dihydrobenzofuran-5-sulfonamide and N-(2,4-dichlorobenzoyl)-5-bromothiophene-2-sulfonamide, or a pharmacologically acceptable salt thereof or a solvate thereof.

**38.** The method according to Claim 33, wherein the sulfonamide compound is sodium salt of N-(2,4-dichlorobenzoyl)-5-bromothiophene-2-sulfonamide.

**39.** A pharmaceutical composition comprising a sulfonamide compound for administering to a patient in combination with Bevacizumab, wherein the sulfonamide compound is at least one compound selected from the group consisting of:

a compound represented by General Formula (I)

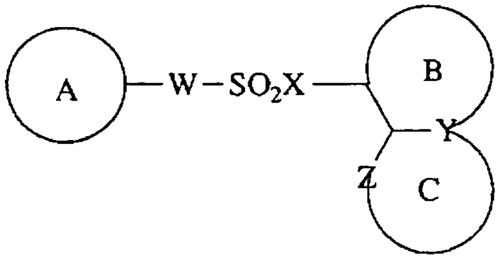

(I)

[wherein, ring A represents an optionally substituted monocyclic or bicyclic aromatic ring,
ring B represents an optionally substituted 6-membered cyclic unsaturated hydrocarbon or 6-membered unsaturated heterocycle containing a nitrogen atom as a heteroatom,
ring C represents an optionally substituted 5-membered heterocycle containing one or two nitrogen atoms,
W represents a single bond or -CH=CH-,
X represents $-N(R^1)-$ or an oxygen atom,
Y represents

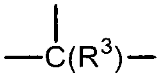 or 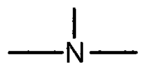 ,

Z represents $-N(R^2)-$,
wherein, $R^1$, $R^2$ and $R^3$ independently represent, identically or differently, a hydrogen atom or a lower alkyl group];
a compound represented by General Formula (II)

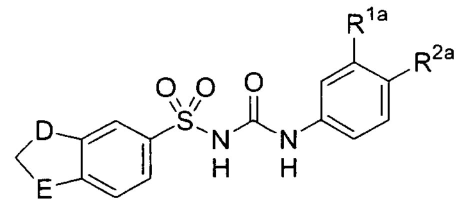

(II)

[wherein, E represents -O-, $-N(CH_3)-$, $-CH_2-$, $-CH_2CH_2-$ or $-CH_2O-$, D represents $-CH_2-$ or -O-, $R^{1a}$ represents a hydrogen atom or a halogen atom, and $R^{2a}$ represents a halogen atom or a trifluoromethyl group];
a compound represented by General Formula (III)

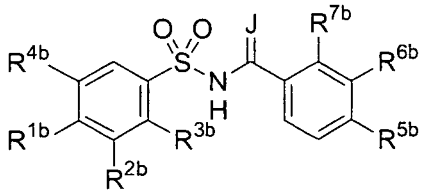

(III)

[wherein, J represents -O- or -NH-, $R^{1b}$ represents a hydrogen atom, a halogen atom, an optionally substituted $C_1$-$C_6$ alkyl group, an optionally substituted $C_1$-$C_4$ alkoxy group, an optionally substituted $C_1$-$C_4$ alkylthio group, $-CF_3$, $-OCF_3$, $-SCF_3$, an optionally substituted $C_1$-$C_4$ alkoxy carbonyl group, a nitro group, an azido group, $-O(SO_2)CH_3$, $-N(CH_3)_2$, a hydroxyl group, a phenyl group, a substituted phenyl group, a pyridinyl group, a thienyl group, a furyl group, a quinolinyl group or a triazole group, $R^{2b}$ represents a hydrogen atom, a halogen atom,

a cyano group, $-CF_3$, an optionally substituted $C_1$-$C_6$ alkyl group, an optionally substituted $C_1$-$C_4$ alkoxy carbonyl group, an optionally substituted $C_1$-$C_4$ alkoxy group, an optionally substituted phenyl group or an optionally substituted quinolinyl group, $R^{3b}$ represents a hydrogen atom or an optionally substituted $C_1$-$C_4$ alkoxy group, $R^{4b}$ represents a hydrogen atom or an optionally substituted $C_1$-$C_6$ alkyl group (provided that at least one of $R^{3b}$ and $R^{4b}$ is a hydrogen atom), $R^{5b}$ represents a hydrogen atom, a halogen atom, an optionally substituted $C_1$-$C_6$ alkyl group, $-CF_3$ or a nitro group, $R^{6b}$ represents a hydrogen atom, a halogen atom or an optionally substituted $C_1$-$C_6$ alkyl group (provided that when $R^{6b}$ is an optionally substituted $C_1$-$C_6$ alkyl group, $R^{5b}$ is a hydrogen atom and $R^{7b}$ is a halogen atom), $R^{7b}$ represents a halogen atom, an optionally substituted $C_1$-$C_6$ alkyl group or $-CF_3$ (provided that when either $R^{5b}$ or $R^{7b}$ is an optionally substituted $C_1$-$C_6$ alkyl group or when $R^{7b}$ is a halogen atom or an optionally substituted $C_1$-$C_6$ alkyl group, either $R^{5b}$ or $R^{6b}$ is a hydrogen atom)];

a compound represented by Formula (IV)

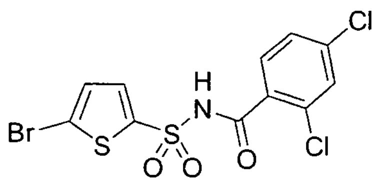

(IV)

;

and

a compound represented by Formula (V)

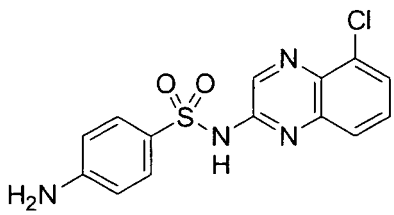

(V)

,

or a pharmacologically acceptable salt thereof or a solvate thereof.

**40.** The pharmaceutical composition according to Claim 39, wherein the sulfonamide compound is at least one compound selected from the group consisting of: N-(3-chloro-1H-indole-7-yl)-4-sulfamoylbenzenesulfonamide, N-(3-cyano-4-methyl-1H-indole-7-yl)-3-cyanobenzenesulfonamide, N-[[(4-chlorophenyl)amino]carbonyl]-2,3-dihydro-1H-indene-5-sulfonamide, N-[[(3,4-dichlorophenyl)amino]carbonyl]-2,3-dihydrobenzofuran-5-sulfonamide, N-(2,4-dichlorobenzoyl)-4-chlorophenylsulfonamide, N-(2,4-dichlorobenzoyl)-5-bromothiophene-2-sulfonamide and 2-sulfanylamide-5-chloroquinoxaline, or a pharmacologically acceptable salt thereof or a solvate thereof.

**41.** The pharmaceutical composition according to Claim 39, wherein the sulfonamide compound is N-(3-cyano-4-methyl-1H-indole-7-yl)-3-cyanobenzenesulfonamide, a pharmacologically acceptable salt thereof or a solvate thereof.

**42.** The pharmaceutical composition according to Claim 39, wherein the sulfonamide compound is N-(3-chloro-1H-indole-7-yl)-4-sulfamoylbenzenesulfonamide, a pharmacologically acceptable salt thereof or a solvate thereof.

**43.** The pharmaceutical composition according to Claim 39, wherein the sulfonamide compound is at least one compound selected from the group consisting of:

N-[[(3,4-dichlorophenyl)amino]carbonyl]-2,3-dihydrobenzofuran-5-sulfonamide; and
N-(2,4-dichlorobenzoyl)-5-bromothiophene-2-sulfonamide, or a pharmacologically acceptable salt thereof or a solvate thereof.

**44.** The pharmaceutical composition according to Claim 39, wherein the sulfonamide compound is sodium salt of N-(2,4-dichlorobenzoyl)-5-bromothiophene-2-sulfonamide.

**45.** The pharmaceutical composition according to any one of Claims 39 to 44, wherein the pharmaceutical composition is used for treating cancer.

**46.** The pharmaceutical composition according to any one of Claims 39 to 44, wherein the pharmaceutical composition is used for inhibiting angiogenesis.

Figure 1

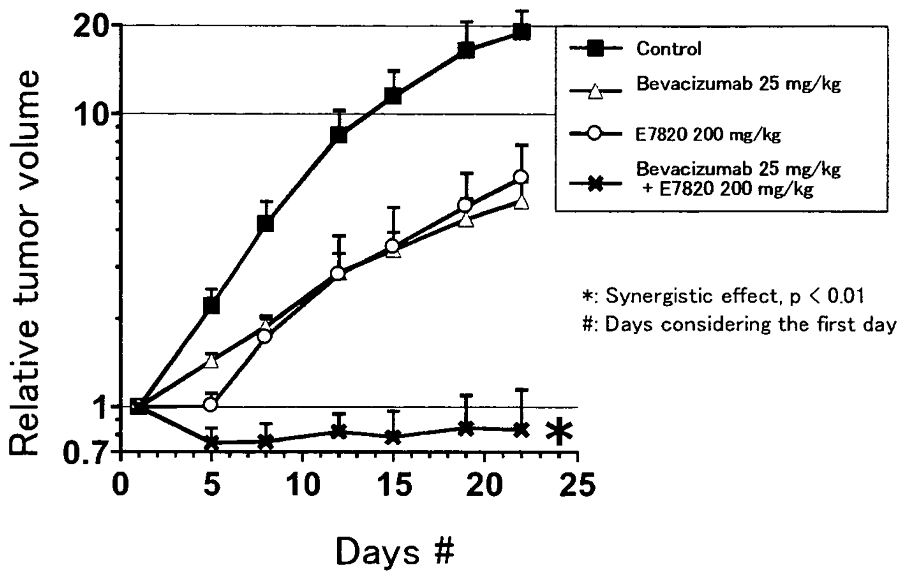


*: Synergistic effect, p < 0.01
#: Days considering the first day of administration as Day 1

Figure 2

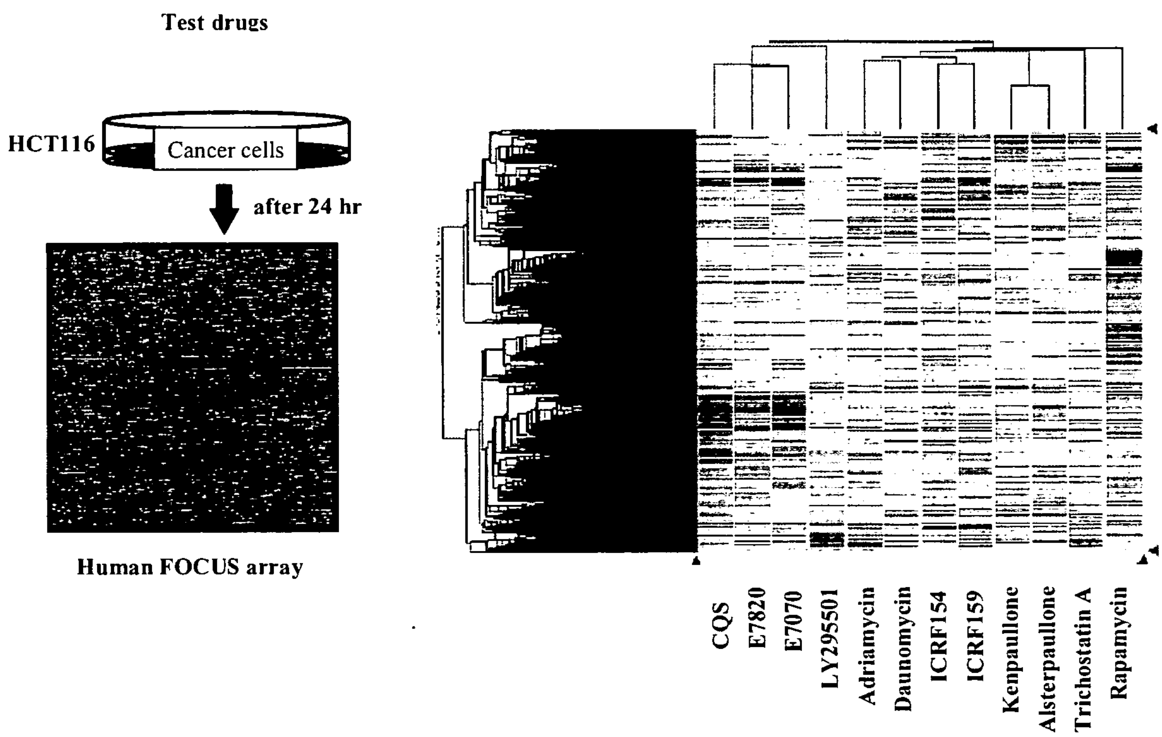

Test drugs
HCT116
Cancer cells
after 24 hr
Human FOCUS array
CQS
E7820
E7070
LY295501
Adriamycin
Daunomycin
ICRF154
ICRF159
Kenpaullone
Alsterpaullone
Trichostatin A
Rapamycin

Figure 3

| | E7070-1 | E7070-2 | E7820-1 | E7820-2 | CQS-1 | CQS-2 | LY1-1 | LY1-2 | LY2-1 | LY2-2 | LY5-1 | LY5-2 | CAI-1 | CAI-2 | Cap-1 | Cap-2 | MST-1 | MST-2 | Etop-1 | Etop-2 | TSA-1 | TSA-2 | Kenp-1 | Kenp-2 |
|---|---|---|---|---|---|---|---|---|---|---|---|---|---|---|---|---|---|---|---|---|---|---|---|---|
| E7070-1 | *1.00* | 0.95 | 0.87 | 0.87 | 0.87 | 0.87 | 0.83 | 0.83 | 0.86 | 0.86 | 0.88 | 0.88 | 0.16 | 0.17 | 0.11 | 0.11 | 0.35 | 0.35 | 0.16 | 0.17 | 0.31 | 0.31 | 0.32 | 0.32 |
| E7070-2 | 0.95 | *1.00* | 0.87 | 0.86 | 0.87 | 0.87 | 0.83 | 0.83 | 0.86 | 0.85 | 0.88 | 0.88 | 0.17 | 0.17 | 0.11 | 0.11 | 0.36 | 0.36 | 0.16 | 0.17 | 0.32 | 0.32 | 0.32 | 0.33 |
| E7820-1 | 0.87 | 0.87 | *1.00* | 0.96 | 0.90 | 0.90 | 0.88 | 0.88 | 0.83 | 0.82 | 0.88 | 0.88 | 0.09 | 0.09 | 0.07 | 0.07 | 0.26 | 0.26 | 0.08 | 0.08 | 0.24 | 0.24 | 0.20 | 0.21 |
| E7820-2 | 0.87 | 0.86 | 0.96 | *1.00* | 0.90 | 0.90 | 0.88 | 0.88 | 0.83 | 0.82 | 0.88 | 0.88 | 0.09 | 0.09 | 0.07 | 0.07 | 0.27 | 0.26 | 0.08 | 0.09 | 0.24 | 0.25 | 0.21 | 0.21 |
| CQS-1 | 0.87 | 0.87 | 0.90 | 0.90 | *1.00* | 0.96 | 0.87 | 0.87 | 0.84 | 0.83 | 0.90 | 0.90 | 0.13 | 0.14 | 0.10 | 0.10 | 0.37 | 0.36 | 0.16 | 0.16 | 0.32 | 0.32 | 0.29 | 0.29 |
| CQS-2 | 0.87 | 0.87 | 0.90 | 0.90 | 0.96 | *1.00* | 0.87 | 0.87 | 0.84 | 0.83 | 0.90 | 0.90 | 0.14 | 0.14 | 0.10 | 0.10 | 0.37 | 0.37 | 0.16 | 0.16 | 0.32 | 0.33 | 0.29 | 0.29 |
| LY1-1 | 0.83 | 0.83 | 0.88 | 0.88 | 0.87 | 0.87 | *1.00* | 0.97 | 0.82 | 0.81 | 0.89 | 0.89 | 0.25 | 0.25 | 0.24 | 0.24 | 0.30 | 0.30 | 0.12 | 0.13 | 0.25 | 0.26 | 0.25 | 0.25 |
| LY1-2 | 0.83 | 0.83 | 0.88 | 0.88 | 0.87 | 0.87 | 0.97 | *1.00* | 0.82 | 0.81 | 0.89 | 0.88 | 0.25 | 0.25 | 0.24 | 0.25 | 0.30 | 0.30 | 0.12 | 0.13 | 0.25 | 0.26 | 0.25 | 0.26 |
| LY2-1 | 0.86 | 0.86 | 0.83 | 0.83 | 0.84 | 0.84 | 0.82 | 0.82 | *1.00* | 0.94 | 0.85 | 0.85 | 0.20 | 0.20 | 0.14 | 0.15 | 0.35 | 0.35 | 0.17 | 0.18 | 0.34 | 0.34 | 0.32 | 0.32 |
| LY2-2 | 0.86 | 0.85 | 0.82 | 0.82 | 0.83 | 0.83 | 0.81 | 0.81 | 0.94 | *1.00* | 0.84 | 0.84 | 0.19 | 0.19 | 0.15 | 0.15 | 0.35 | 0.35 | 0.18 | 0.18 | 0.33 | 0.34 | 0.31 | 0.32 |
| LY5-1 | 0.88 | 0.88 | 0.88 | 0.88 | 0.90 | 0.90 | 0.89 | 0.89 | 0.85 | 0.84 | *1.00* | 0.97 | 0.20 | 0.20 | 0.15 | 0.15 | 0.39 | 0.39 | 0.21 | 0.22 | 0.34 | 0.34 | 0.31 | 0.32 |
| LY5-2 | 0.88 | 0.88 | 0.88 | 0.88 | 0.90 | 0.90 | 0.89 | 0.88 | 0.85 | 0.84 | 0.97 | *1.00* | 0.20 | 0.20 | 0.15 | 0.15 | 0.39 | 0.39 | 0.21 | 0.22 | 0.34 | 0.34 | 0.31 | 0.32 |
| CAI-1 | 0.16 | 0.17 | 0.09 | 0.09 | 0.13 | 0.14 | 0.25 | 0.25 | 0.20 | 0.19 | 0.20 | 0.20 | *1.00* | 0.98 | 0.75 | 0.75 | 0.38 | 0.38 | 0.34 | 0.34 | 0.25 | 0.25 | 0.25 | 0.25 |
| CAI-2 | 0.17 | 0.17 | 0.09 | 0.09 | 0.14 | 0.14 | 0.25 | 0.25 | 0.20 | 0.19 | 0.20 | 0.20 | 0.98 | *1.00* | 0.75 | 0.75 | 0.38 | 0.38 | 0.34 | 0.33 | 0.25 | 0.25 | 0.26 | 0.26 |
| Cap-1 | 0.11 | 0.11 | 0.07 | 0.07 | 0.10 | 0.10 | 0.24 | 0.24 | 0.14 | 0.15 | 0.15 | 0.15 | 0.75 | 0.75 | *1.00* | 0.99 | 0.35 | 0.34 | 0.31 | 0.31 | 0.19 | 0.19 | 0.14 | 0.14 |
| Cap-2 | 0.11 | 0.11 | 0.07 | 0.07 | 0.10 | 0.10 | 0.24 | 0.25 | 0.15 | 0.15 | 0.15 | 0.15 | 0.75 | 0.75 | 0.99 | *1.00* | 0.34 | 0.34 | 0.31 | 0.31 | 0.18 | 0.18 | 0.14 | 0.14 |
| MST-1 | 0.35 | 0.36 | 0.26 | 0.27 | 0.37 | 0.37 | 0.30 | 0.30 | 0.35 | 0.35 | 0.39 | 0.39 | 0.38 | 0.38 | 0.35 | 0.34 | *1.00* | 0.94 | 0.62 | 0.62 | 0.57 | 0.57 | 0.39 | 0.39 |
| MST-2 | 0.35 | 0.36 | 0.26 | 0.26 | 0.36 | 0.37 | 0.30 | 0.30 | 0.35 | 0.35 | 0.39 | 0.39 | 0.38 | 0.38 | 0.34 | 0.34 | 0.94 | *1.00* | 0.62 | 0.63 | 0.57 | 0.57 | 0.39 | 0.39 |
| Etop-1 | 0.16 | 0.16 | 0.08 | 0.08 | 0.16 | 0.16 | 0.12 | 0.12 | 0.17 | 0.18 | 0.21 | 0.21 | 0.34 | 0.34 | 0.31 | 0.31 | 0.62 | 0.62 | *1.00* | 0.96 | 0.45 | 0.45 | 0.24 | 0.24 |
| Etop-2 | 0.17 | 0.17 | 0.08 | 0.09 | 0.16 | 0.16 | 0.13 | 0.13 | 0.18 | 0.18 | 0.22 | 0.22 | 0.34 | 0.33 | 0.31 | 0.31 | 0.62 | 0.63 | 0.96 | *1.00* | 0.45 | 0.45 | 0.24 | 0.24 |
| TSA-1 | 0.31 | 0.32 | 0.24 | 0.24 | 0.32 | 0.32 | 0.25 | 0.25 | 0.34 | 0.33 | 0.34 | 0.34 | 0.25 | 0.25 | 0.19 | 0.18 | 0.57 | 0.57 | 0.45 | 0.45 | *1.00* | 0.93 | 0.34 | 0.34 |
| TSA-2 | 0.31 | 0.32 | 0.24 | 0.25 | 0.32 | 0.33 | 0.26 | 0.26 | 0.34 | 0.34 | 0.34 | 0.34 | 0.25 | 0.25 | 0.19 | 0.18 | 0.57 | 0.57 | 0.45 | 0.45 | 0.93 | *1.00* | 0.34 | 0.34 |
| Kenp-1 | 0.32 | 0.32 | 0.20 | 0.21 | 0.29 | 0.29 | 0.25 | 0.25 | 0.32 | 0.31 | 0.31 | 0.31 | 0.25 | 0.26 | 0.14 | 0.14 | 0.39 | 0.39 | 0.24 | 0.24 | 0.34 | 0.34 | *1.00* | 0.96 |
| Kenp-2 | 0.32 | 0.33 | 0.21 | 0.21 | 0.29 | 0.29 | 0.25 | 0.26 | 0.32 | 0.32 | 0.32 | 0.32 | 0.25 | 0.26 | 0.14 | 0.14 | 0.39 | 0.39 | 0.24 | 0.24 | 0.34 | 0.34 | 0.96 | *1.00* |

0.7<X<=1.0

0.4<X<=0.7

0<X<=0.4

Figure 4

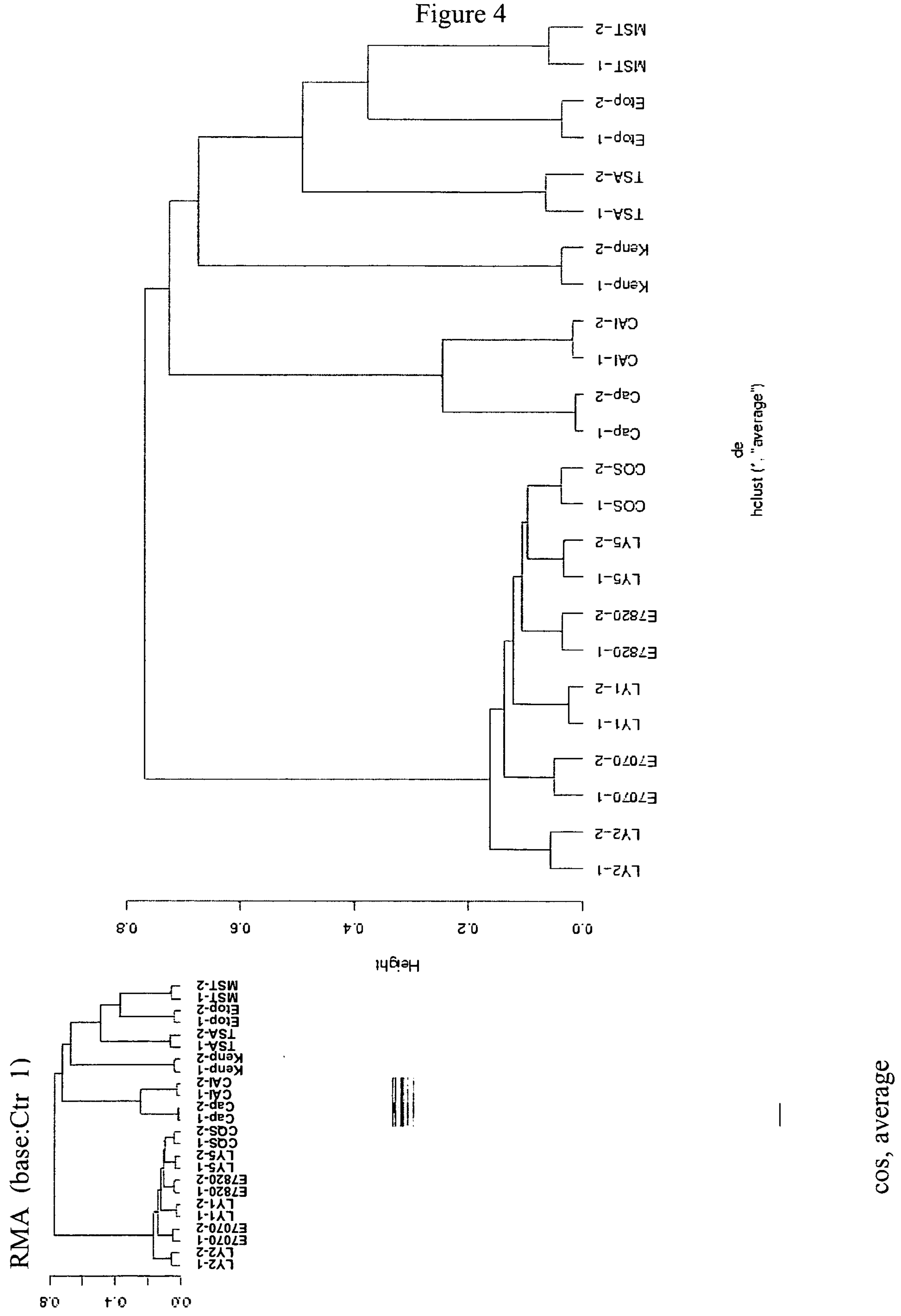
MST-2
MST-1
Etop-2
Etop-1
TSA-2
TSA-1
Kenp-2
Kenp-1
CAI-2
CAI-1
Cap-2
Cap-1
COS-2
COS-1
LY5-2
LY5-1
E7820-2
E7820-1
LY1-2
LY1-1
E7070-2
E7070-1
LY2-2
LY2-1
0.0
0.2
0.4
0.6
0.8
Height
de
hclust (*, "average")
RMA (base:Ctr 1)
cos, average

Figure 5

| | E7070-1 | E7070-2 | E7820-1 | E7820-2 | CQS-1 | CQS-2 | LY1-1 | LY1-2 | LY2-1 | LY2-2 | LY5-1 | LY5-2 | CAI-1 | CAI-2 | Cap-1 | Cap-2 | MST-1 | MST-2 | Etop-1 | Etop-2 | TSA-1 | TSA-2 | Kenp-1 | Kenp-2 |
|---|---|---|---|---|---|---|---|---|---|---|---|---|---|---|---|---|---|---|---|---|---|---|---|---|
| E7070-1 | *1.00* | 0.94 | 0.87 | 0.87 | 0.86 | 0.86 | 0.81 | 0.81 | 0.84 | 0.83 | 0.86 | 0.86 | 0.04 | 0.04 | 0.03 | 0.04 | 0.24 | 0.23 | 0.06 | 0.06 | 0.22 | 0.22 | 0.19 | 0.19 |
| E7070-2 | 0.94 | *1.00* | 0.86 | 0.86 | 0.85 | 0.85 | 0.81 | 0.81 | 0.84 | 0.83 | 0.86 | 0.86 | 0.04 | 0.04 | 0.03 | 0.03 | 0.24 | 0.23 | 0.05 | 0.06 | 0.22 | 0.22 | 0.19 | 0.20 |
| E7820-1 | 0.87 | 0.86 | *1.00* | 0.96 | 0.90 | 0.90 | 0.86 | 0.86 | 0.81 | 0.80 | 0.87 | 0.87 | 0.00 | 0.00 | 0.02 | 0.03 | 0.22 | 0.22 | 0.03 | 0.04 | 0.21 | 0.22 | 0.12 | 0.12 |
| E7820-2 | 0.87 | 0.86 | 0.96 | *1.00* | 0.90 | 0.90 | 0.85 | 0.85 | 0.81 | 0.80 | 0.87 | 0.87 | 0.00 | 0.00 | 0.02 | 0.03 | 0.22 | 0.21 | 0.03 | 0.04 | 0.21 | 0.22 | 0.12 | 0.13 |
| CQS-1 | 0.86 | 0.85 | 0.90 | 0.90 | *1.00* | 0.96 | 0.85 | 0.85 | 0.82 | 0.81 | 0.89 | 0.88 | 0.02 | 0.02 | 0.03 | 0.03 | 0.28 | 0.27 | 0.07 | 0.07 | 0.25 | 0.25 | 0.17 | 0.18 |
| CQS-2 | 0.86 | 0.85 | 0.90 | 0.90 | 0.96 | *1.00* | 0.86 | 0.85 | 0.82 | 0.81 | 0.89 | 0.89 | 0.02 | 0.02 | 0.03 | 0.04 | 0.28 | 0.27 | 0.07 | 0.07 | 0.25 | 0.25 | 0.17 | 0.18 |
| LY1-1 | 0.81 | 0.81 | 0.86 | 0.85 | 0.85 | 0.86 | *1.00* | 0.97 | 0.80 | 0.79 | 0.88 | 0.88 | 0.17 | 0.17 | 0.19 | 0.19 | 0.20 | 0.20 | 0.04 | 0.04 | 0.17 | 0.17 | 0.15 | 0.16 |
| LY1-2 | 0.81 | 0.81 | 0.86 | 0.85 | 0.85 | 0.85 | 0.97 | *1.00* | 0.80 | 0.79 | 0.87 | 0.87 | 0.17 | 0.17 | 0.19 | 0.20 | 0.20 | 0.20 | 0.03 | 0.04 | 0.17 | 0.17 | 0.15 | 0.16 |
| LY2-1 | 0.84 | 0.84 | 0.81 | 0.81 | 0.82 | 0.82 | 0.80 | 0.80 | *1.00* | 0.93 | 0.83 | 0.83 | 0.09 | 0.09 | 0.07 | 0.08 | 0.23 | 0.23 | 0.07 | 0.08 | 0.24 | 0.24 | 0.20 | 0.20 |
| LY2-2 | 0.83 | 0.83 | 0.80 | 0.80 | 0.81 | 0.81 | 0.79 | 0.79 | 0.93 | *1.00* | 0.82 | 0.82 | 0.08 | 0.08 | 0.07 | 0.08 | 0.23 | 0.22 | 0.07 | 0.07 | 0.23 | 0.24 | 0.19 | 0.20 |
| LY5-1 | 0.86 | 0.86 | 0.87 | 0.87 | 0.89 | 0.89 | 0.88 | 0.87 | 0.83 | 0.82 | *1.00* | 0.96 | 0.09 | 0.09 | 0.08 | 0.08 | 0.28 | 0.28 | 0.11 | 0.12 | 0.24 | 0.24 | 0.19 | 0.20 |
| LY5-2 | 0.86 | 0.86 | 0.87 | 0.87 | 0.88 | 0.89 | 0.88 | 0.87 | 0.83 | 0.82 | 0.96 | *1.00* | 0.09 | 0.09 | 0.07 | 0.08 | 0.28 | 0.28 | 0.11 | 0.12 | 0.24 | 0.24 | 0.19 | 0.20 |
| CAI-1 | 0.04 | 0.04 | 0.00 | 0.00 | 0.02 | 0.02 | 0.17 | 0.17 | 0.09 | 0.08 | 0.09 | 0.09 | *1.00* | 0.98 | 0.74 | 0.74 | 0.26 | 0.26 | 0.25 | 0.25 | 0.13 | 0.13 | 0.14 | 0.14 |
| CAI-2 | 0.04 | 0.04 | 0.00 | 0.00 | 0.02 | 0.02 | 0.17 | 0.17 | 0.09 | 0.08 | 0.09 | 0.09 | 0.98 | *1.00* | 0.74 | 0.74 | 0.26 | 0.26 | 0.25 | 0.25 | 0.13 | 0.13 | 0.15 | 0.15 |
| Cap-1 | 0.03 | 0.03 | 0.02 | 0.02 | 0.03 | 0.03 | 0.19 | 0.19 | 0.07 | 0.07 | 0.08 | 0.07 | 0.74 | 0.74 | *1.00* | 0.99 | 0.28 | 0.28 | 0.26 | 0.26 | 0.12 | 0.12 | 0.06 | 0.06 |
| Cap-2 | 0.04 | 0.03 | 0.03 | 0.03 | 0.03 | 0.04 | 0.19 | 0.20 | 0.08 | 0.08 | 0.08 | 0.08 | 0.74 | 0.74 | 0.99 | *1.00* | 0.28 | 0.28 | 0.26 | 0.25 | 0.11 | 0.12 | 0.06 | 0.06 |
| MST-1 | 0.24 | 0.24 | 0.22 | 0.22 | 0.28 | 0.28 | 0.20 | 0.20 | 0.23 | 0.23 | 0.28 | 0.28 | 0.26 | 0.26 | 0.28 | 0.28 | *1.00* | 0.92 | 0.57 | 0.57 | 0.50 | 0.50 | 0.24 | 0.25 |
| MST-2 | 0.23 | 0.23 | 0.22 | 0.21 | 0.27 | 0.27 | 0.20 | 0.20 | 0.23 | 0.22 | 0.28 | 0.28 | 0.26 | 0.26 | 0.28 | 0.28 | 0.92 | *1.00* | 0.57 | 0.57 | 0.50 | 0.50 | 0.24 | 0.25 |
| Etop-1 | 0.06 | 0.05 | 0.03 | 0.03 | 0.07 | 0.07 | 0.04 | 0.03 | 0.07 | 0.07 | 0.11 | 0.11 | 0.25 | 0.25 | 0.26 | 0.26 | 0.57 | 0.57 | *1.00* | 0.96 | 0.39 | 0.39 | 0.12 | 0.13 |
| Etop-2 | 0.06 | 0.06 | 0.04 | 0.04 | 0.07 | 0.07 | 0.04 | 0.04 | 0.08 | 0.07 | 0.12 | 0.12 | 0.25 | 0.25 | 0.26 | 0.25 | 0.57 | 0.57 | 0.96 | *1.00* | 0.39 | 0.39 | 0.12 | 0.13 |
| TSA-1 | 0.22 | 0.22 | 0.21 | 0.21 | 0.25 | 0.25 | 0.17 | 0.17 | 0.24 | 0.23 | 0.24 | 0.24 | 0.13 | 0.13 | 0.12 | 0.11 | 0.50 | 0.50 | 0.39 | 0.39 | *1.00* | 0.93 | 0.21 | 0.22 |
| TSA-2 | 0.22 | 0.22 | 0.22 | 0.22 | 0.25 | 0.25 | 0.17 | 0.17 | 0.24 | 0.24 | 0.24 | 0.24 | 0.13 | 0.13 | 0.12 | 0.12 | 0.50 | 0.50 | 0.39 | 0.39 | 0.93 | *1.00* | 0.22 | 0.22 |
| Kenp-1 | 0.19 | 0.19 | 0.12 | 0.12 | 0.17 | 0.17 | 0.15 | 0.15 | 0.20 | 0.19 | 0.19 | 0.19 | 0.14 | 0.15 | 0.06 | 0.06 | 0.24 | 0.24 | 0.12 | 0.12 | 0.21 | 0.22 | *1.00* | 0.95 |
| Kenp-2 | 0.19 | 0.20 | 0.12 | 0.13 | 0.18 | 0.18 | 0.16 | 0.16 | 0.20 | 0.20 | 0.20 | 0.20 | 0.14 | 0.15 | 0.06 | 0.06 | 0.25 | 0.25 | 0.13 | 0.13 | 0.22 | 0.22 | 0.95 | *1.00* |

0.7<X<=1.0 (bold solid box)

0.4<X<=0.7 (dashed box)

0<X<=0.4 (thin box)

Figure 6

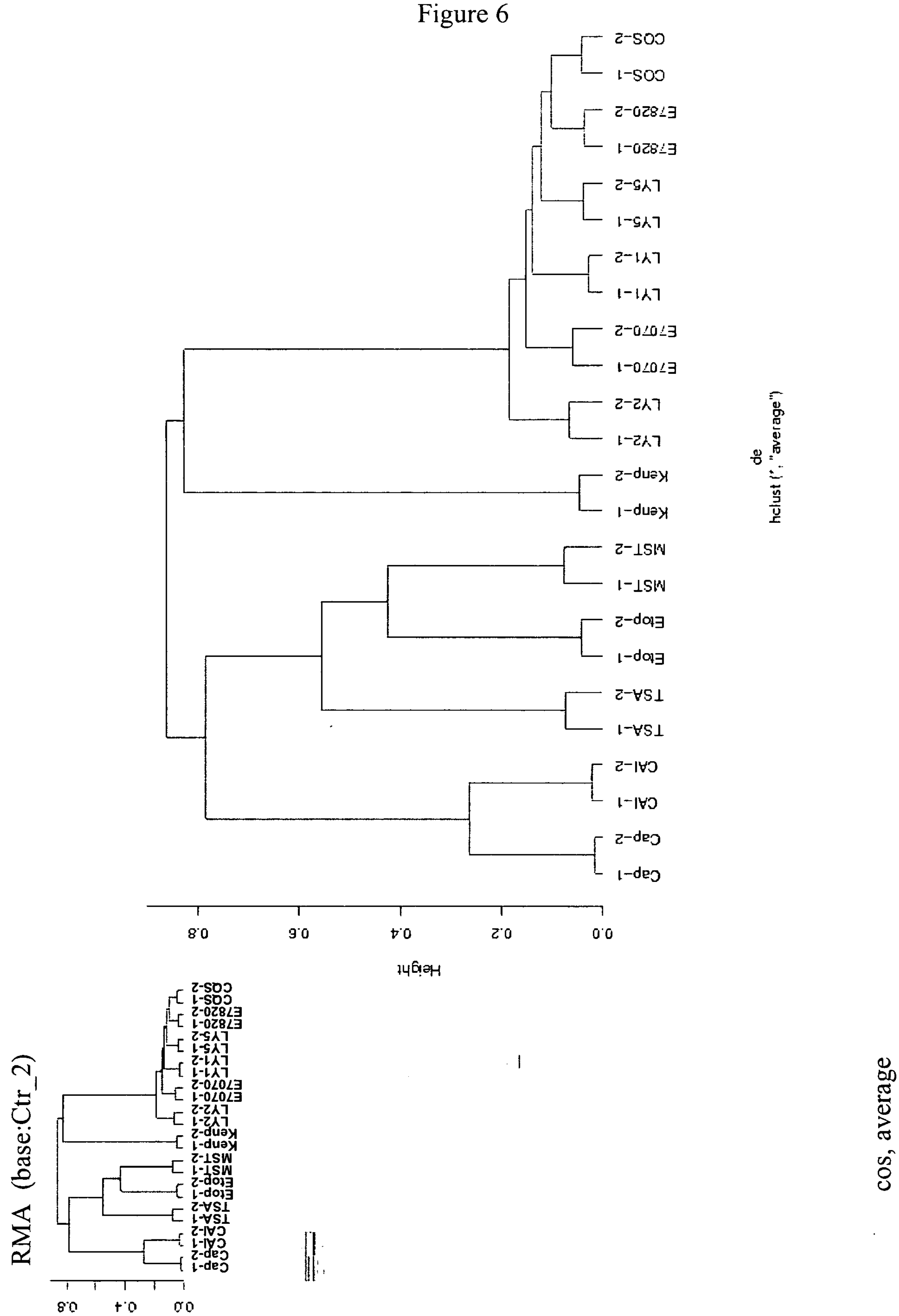
RMA (base:Ctr_2)
cos, average
Height
hclust (*, "average")
de
COS-2
COS-1
E7820-2
E7820-1
LY5-2
LY5-1
LY1-2
LY1-1
E7070-2
E7070-1
LY2-2
LY2-1
Kenp-2
Kenp-1
MST-2
MST-1
Etop-2
Etop-1
TSA-2
TSA-1
CAI-2
CAI-1
Cap-2
Cap-1
0.0
0.2
0.4
0.6
0.8

Figure 7

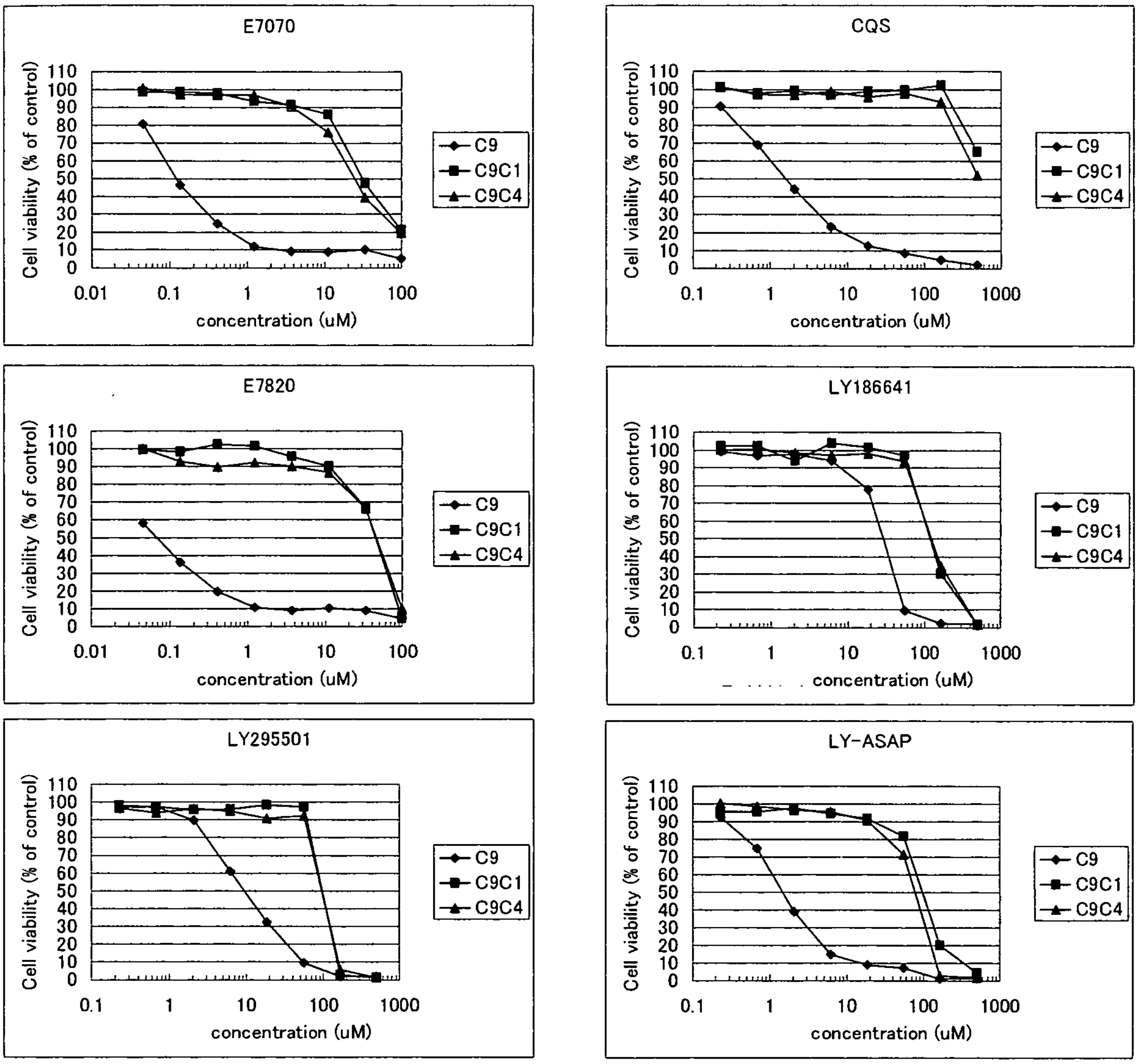
E7070
CQS
E7820
LY186641
LY295501
LY-ASAP
Cell viability (% of control)
concentration (uM)
C9
C9C1
C9C4

Figure 8

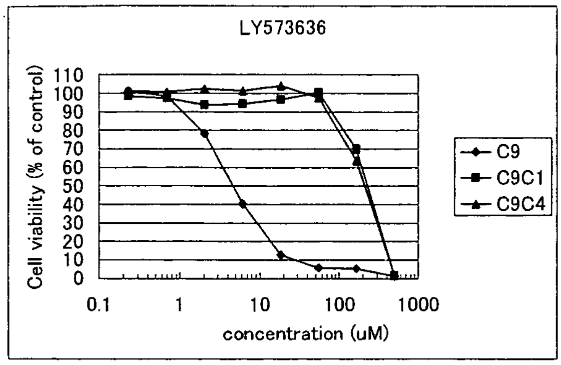
LY573636
Cell viability (% of control)
110
100
90
80
70
60
50
40
30
20
10
0
0.1
1
10
100
1000
concentration (uM)
C9
C9C1
C9C4

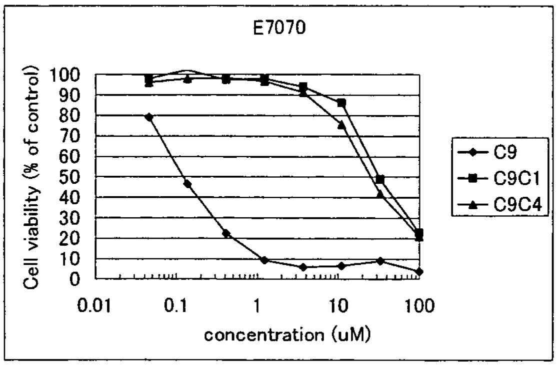
E7070
Cell viability (% of control)
100
90
80
70
60
50
40
30
20
10
0
0.01
0.1
1
10
100
concentration (uM)
C9
C9C1
C9C4

Figure 9

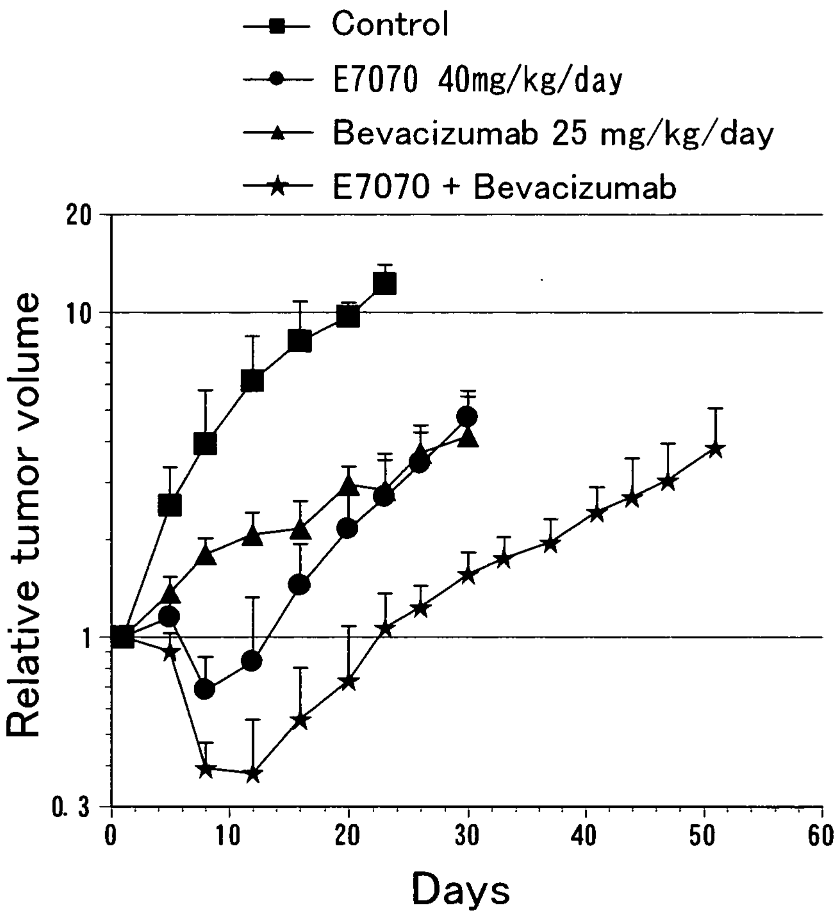
Control
E7070 40mg/kg/day
Bevacizumab 25 mg/kg/day
E7070 + Bevacizumab
Relative tumor volume
20
10
1
0.3
0
10
20
30
40
50
60
Days

**INTERNATIONAL SEARCH REPORT**

International application No.
PCT/JP2006/304208

A. CLASSIFICATION OF SUBJECT MATTER
***A61K39/395***(2006.01), ***A61K31/18***(2006.01), ***A61K31/404***(2006.01), ***A61K31/381***(2006.01), ***A61K31/498***(2006.01), ***A61K31/64***(2006.01), ***A61P9/00***(2006.01), ***A61P35/00***(2006.01), *C07D209/30*(2006.01), *C07D209/42*(2006.01), *C07D241/44*

According to International Patent Classification (IPC) or to both national classification and IPC

B. FIELDS SEARCHED

Minimum documentation searched (classification system followed by classification symbols)
A61K31/18, A61K31/381, A61K31/404, A61K31/498, A61K31/64, A61K39/395, A61P9/00, A61P35/00, C07D209/30, C07D209/42, C07D241/44, C07D333/34

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
CA(STN), MEDLINE(STN), EMBASE(STN), BIOSIS(STN)

C. DOCUMENTS CONSIDERED TO BE RELEVANT

| Category* | Citation of document, with indication, where appropriate, of the relevant passages | Relevant to claim No. |
|---|---|---|
| A | WO 2003/074045 A1 (Eisai Co., Ltd.), 12 September, 2003 (12.09.03), Claims; examples & AU 2003211594 A1 & EP 1481678 A1 & US 2005/0119303 A1 | 1-32,39-46 |
| A | PRESTA, L.G. et. al., Humanization of an Anti-vascular Endothelial Growth Factor Monoclonal Antibody for the Therapy of Solid Tummors and Other Disorders., Cancer.Res., 1997, Vol.57, No.20, pages 4593 to 4599, full text | 1-32,39-46 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 May, 2006 (24.05.06) | 06 June, 2006 (06.06.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.
PCT/JP2006/304208

C (Continuation). DOCUMENTS CONSIDERED TO BE RELEVANT

| Category* | Citation of document, with indication, where appropriate, of the relevant passages | Relevant to claim No. |
|---|---|---|
| A | JP 07-165708 A (Eisai Co., Ltd.), 27 June, 1995 (27.06.95), Example 19<br>& WO 1995/007276 A1 & AU 9476237 A<br>& NO 9501813 A & FI 9502272 A<br>& EP 673937 A & NZ 273073 A<br>& CN 1114506 A & AU 9717785 A<br>& US 5721246 A & US 5767283 A | 2,4,10,12, 18,20,26,28, 40,42 |
| A | EP 0222475 A1 (ELI LILLY AND CO.), 17 September, 1986 (17.09.86), Example 1<br>& AU 8662942 A & JP 62-096459 A<br>& DK 8604469 A & PT 83389A<br>& ZA 8607011 A & CN 8606428 A<br>& ES 2001698 A & SU 1545939 A<br>& IL 80032 A | 2,10,18,26, 40 |
| A | WO 2002/098848 A1 (ELI LILLY AND CO.), 12 December, 2002 (12.12.02), Example 111<br>& NO 200305366 A & EP 1401806 A1<br>& KR 2004007656 A & BR 200210078 A<br>& AU 2002259204 A1 & US 2004/0157741 A1<br>& JP 2004-530709 A & CN 1514824 A<br>& MX 2003011197 A1 & ZA 200308644 A<br>& NZ 529098 A | 2,10,18,26, 40 |
| A | WO 2003/035629 A1 (ELI LILLY AND CO.), 01 May, 2003 (01.05.03), Examples 63, 64<br>& EP 1442030 A1 & BR 200212386 A<br>& AU 2002334817 A & US 2004/0198784 A1<br>& KR 2004062583 A & NO 200401316 A<br>& JP 2005-511547 A & CN 1575286 A<br>& MX 2004003886 A1 & ZA 200403089 A | 2,5,6,10,13, 14,18,21,22, 26,29,30,40, 43,44 |
| A | WOLF, F.J. et.al., Substituted Sulfaquinoxalines. III.Extension of the Glyoxalate Synthesis of 2-Aminoquinoxaline., J.Am.Chem.Soc., 1949, Vol.71, pages 6 to 10, full text | 2,10,18,26, 40 |

Form PCT/ISA/210 (continuation of second sheet) (April 2005)

**INTERNATIONAL SEARCH REPORT**

International application No.
PCT/JP2006/304208

Continuation of A. CLASSIFICATION OF SUBJECT MATTER
(International Patent Classification (IPC))

(2006.01), *C07D333/34*(2006.01)

(According to International Patent Classification (IPC) or to both national classification and IPC)

Form PCT/ISA/210 (extra sheet) (April 2005)

**INTERNATIONAL SEARCH REPORT**

International application No.
PCT/JP2006/304208

**Box No. II Observations where certain claims were found unsearchable (Continuation of item 2 of first sheet)**

This international search report has not been established in respect of certain claims under Article 17(2)(a) for the following reasons:

1. [X] Claims Nos.: 33-38
because they relate to subject matter not required to be searched by this Authority, namely:
The inventions as set forth in claims 33 to 38 pertain to methods for treatment of the human body by therapy and thus relate to a subject matter which this International Searching Authority is not required, under the provisions of Article 17(2)(a)(i) of the PCT (continued to extra sheet)

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the

[ ] The additional search fees were accompanied by the applicant's protest and, where applicable, payment of a protest fee..

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (April 2005)

**INTERNATIONAL SEARCH REPORT**

International application No.
PCT/JP2006/304208

Continuation of Box No.II-1 of continuation of first sheet(2)

and Rule 39.1(iv) of the Regulations under the PCT, to search.

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 7165708 A
- WO 0050395 A
- EP 0222475 A
- WO 02098848 A
- WO 2003035629 A
- WO 03074045 A
- WO 9507276 A
- EP 0222475 A1
- EP 0555036 A2


### Non-patent literature cited in the description


- Direct evidence that the VEGF-specific antibody bevacizumab has antivascular effects in human rectal cancer. *Nat Med.,* February 2004, vol. 10 (2), 145-7
- **SCHENA M ; SHALON D ; DAVIS RW ; BROWN PO.** *Science,* 1995, vol. 270, 467-70
- **LOCKHART, D.J ; DONG, H. ; BYRNE, M.C. ; FOLLETTIE, M.T. ; GALLO, M.V. ; CHEE, M.S. ; MITTMANN, M. ; WANG C. ; KOBAYASHI, M. ; HORTON, H.** *Nature Biotechnology,* 1996, vol. 14, 1675-1680
- **RHEE CH ; RUAN S ; CHEN S ; CHENCHIK A ; LEVIN VA ; YUNG AW ; FULLER GN ; ZHANG W.** *Oncol Rep,* 1999, vol. 6, 393-401
- **ZIMMERMANN J ; ERDMANN D ; LALANDE I ; GROSSENBACHER R ; NOORANI M ; FURST P.** *Oncogene,* 2000, vol. 19, 2913-20
- **KUDOH K ; RAMANNA M ; RAVATN R ; ELKAHLOUN AG ; BITTNER ML ; MELTZER PS ; TRENT JM ; DALTON WS ; CHIN KV.** *Cancer Res,* 2000, 4161-6
- **ROSS DT ; SCHERF U ; EISEN MB ; PEROU CM ; REES C ; SPELLMAN P ; IYER V ; JEFFREY SS ; VAN DE RIJN M ; WALTHAM M.** *Nat Genet,* 2000, vol. 24, 227-35
- **SCHERF U ; ROSS DT ; WALTHAM M ; SMITH LH ; LEE JK ; TANABE L ; KOHN KW ; REINHOLD WC ; MYERS TG ; ANDREWS DT.** *Nat Genet,* 2000, vol. 24, 236-44
- *J. Am. Chem. Soc.,* 1947, vol. 71, 6-10
- **CHOU et al.** *Adv. Enzyme Regul.,* 1984, vol. 22, 27-55
- *Biostatistics,* 2003, vol. 4, 249-264
- **PAULL, K. D. et al.** Display and analysis of patterns of differential activity of drugs against human tumor cell lines: development of mean graph and COMPARE algorithm. *J. Natl. Cancer Inst.,* 1989, vol. 81, 1088-1092
- **MONKS, A et al.** Feasibility of a high-flux anticancer drug screen using a diverse panel of cultured human tumor cell lines. *J. Natl. Cancer Inst.,* 1991, vol. 83, 757-766
- *Molecular Cancer Therapeutics,* 2002, vol. 1, 275-286
- **MOSSMANN T.** *J. Immunol. Methods,* 1983, vol. 65, 55-63